# EUROPEAN PATENT APPLICATION

(11) **EP 4 650 362 A1**
(43) Date of publication of application: **19.11.2025**
(21) Application number: 24741229.9
(22) Date of filing: 09.01.2024
(51) Int. Cl.: C07K 14/00, A61K 38/16, A61P 9/10

(54) **NEUROPROTECTIVE PSD-95 POLYPEPTIDE INHIBITOR AND USE THEREOF**

(30) Priority: 09.01.2023 CN 202310027484; 01.09.2023 CN 202311125942
(71) Applicant: Beijing Tuo Jie Biopharmaceutical Co. Ltd., Beijing 102206 (CN)
(72) Inventor: WANG, Qianqian, Beijing 102206 (CN); WU, Fangzhou, Beijing 102206 (CN); HU, Dongmei, Beijing 102206 (CN); QU, Liang, Beijing 102206 (CN); TANG, Jingjing, Beijing 102206 (CN); WANG, Lei, Beijing 102206 (CN)
(74) Representative: Dragotti & Associati S.P.A.
(86) International application number: PCT/CN2024/071297
(87) International publication number: WO 2024/149230

(57) **Abstract**

Provided are a neuroprotective PSD-95 polypeptide inhibitor and the use thereof. Specifically, provided are a series of active peptides that can bind to a PDZ1 domain or PDZ2 domain of PSD-95, and a pharmaceutically acceptable salt and pharmaceutical composition thereof. The active peptide has a structure as represented by formula (I), (I), and can be used as an inhibitor of protein-protein interaction mediated by PSD-95 for the treatment of stroke and nervous system related diseases.

## Description

The present application claims priority to Chinese Patent Application No. CN202310027484.8 filed on January 9, 2023 and Chinese Patent Application No. CN202311125942.8 filed on September 1, 2023.

### TECHNICAL FIELD

The present disclosure pertains to the field of biopharmaceutics and particularly relates to an active peptide capable of binding to the PDZ1 or PDZ2 domain of PSD-95 that can be used as an inhibitor of PSD-95-mediated protein-protein interactions for treating stroke and nervous system-related diseases.

### BACKGROUND

Postsynaptic density protein-95 (PSD-95) is an important scaffold protein located at the postsynaptic membrane of the central nervous system. It consists of three repeat PDZ domains (PDZ1, PDZ2, and PDZ3) at the N-terminus, an SH3 domain in the middle, and a guanylate kinase domain at the C-terminus. PDZ domains are common protein-protein interaction domains. PSD-95 binds to the tSXV-COOH of NMDARs (N-methyl-D-aspartate receptors) through PDZ1 and PDZ2 and binds to the PDZ of nNOS (neuronal nitric oxide synthase) through PDZ2 (Acta Pharmacol. Sin., 2018, 39:661-668).

After an ischemic stroke, local cerebral blood flow disorder leads to glutamic acid accumulation in the extracellular space. The excessively released excitatory glutamic acid persistently acts upon glutamate receptors, resulting in neuronal depolarization and calcium influx. The Ca²⁺ overload activates nNOS through calmodulin, resulting in an abnormal increase in nitric oxide (NO) production and ultimately neuronal damage.

PSD-95 inhibitors can effectively interfere with the intracellular interactions between NMDA receptors and PSD-95. Disruption of the GluN2B-PSD95-nNOS complex can inhibit NMDA-mediated NO production, thereby protecting neurons. Nerinetide (also known as TAT-NR2B9c or NA-1) is a PSD-95 inhibitor developed by the Canadian company NoNO Inc. (Science, 2002, 298:846-50). It is a neuroexcitotoxicity inhibitor. Nerinetide consists of 20 amino acid residues. The TAT is derived from the trans-activator of transcription of human (HIV-1), which can cross various cell membranes. The NR2B9c is selected from the group consisting of nine C-terminal amino acids of the NR2B subunit.

In preclinical animal models (rats and cynomolgus monkeys), administration of nerinetide after ischemic strokes can significantly reduce infarct size, improving neurobehavioral function (Sci. Transl. Med., 2021, 13, eabb1498). Both the results of a phase II clinical study in patients who underwent cerebral endovascular aneurysm repair (ENACT) and the results of a phase III clinical study in patients with severe acute ischemic stroke (AIS) who underwent endovascular thrombectomy (EVT) show that no drug-related serious adverse events were observed, and that the safety profile of nerinetide is comparable to those of placebos.

Currently, studies on PSD-95 inhibitors include WO2015078477A, WO2022150655A, etc. It is still necessary to develop PSD-95 inhibitors with high stability and good pharmacokinetic profiles to meet clinical needs in the treatment of diseases such as ischemic strokes.

### SUMMARY

The present disclosure provides a compound represented by formula (G) or a pharmaceutically acceptable salt thereof:

X₁-X₂-X₃-X₄-X₅-X₆-X₇-X₈-X₉ (G),

wherein X₁ is selected from the group consisting of Gly and absent;
X₂ is selected from the group consisting of Aib, Ala, Ile, Cha, Phe, Trp, 1-Nal, Ser, Lys, Arg, Nle, Nva, Orn, cyclopropyl Ala, 4-thiazolyl Ala, homoLeu, and absent;
X₃ is selected from the group consisting of Dap, Dab, Thr, Aib, Ala, Cha, Phe, Trp, 1-Nal, Asn, Glu, Lys, Arg, 1Me-Trp, 2-Nal, Gln, Thr, 4-thiazolyl Ala, and absent;
X₄ is selected from the group consisting of Thr, homoArg, Ser, Phe, and absent;
X₅ is selected from the group consisting of Tle, homoArg, Arg, Lys, 4-thiazolyl Ala, Chg, Cha, homoPhe, 4F-Phe, 3F-Phe, 2F-Phe, 4-Pal, 3-Pal, 2-Pal, Cit, Orn, and Phe;
X₆ is selected from the group consisting of Tle, Ile, Phe, Trp, Leu, 1-Nal, Nle, D-Nle, D-Trp, and D-Phe;
X₇ is selected from the group consisting of Thr and Ser;
X₈ is Asp, Cha, Leu, Trp, 1-Nal, Phe, Val, Ala, Abu, Ser, or Thr;
X₉ is Val.

In another aspect, the present disclosure provides a compound represented by formula (I) or a pharmaceutically acceptable salt thereof:

CPP-L₁-X₁-X₂-X₃-X₄-X₅-X₆-X₇-X₈-X₉ (I)

wherein X₁ is selected from the group consisting of Gly and absent;
X₁ is selected from the group consisting of Gly and absent;
X₂ is selected from the group consisting of Aib, Ala, Ile, Cha, Phe, Trp, 1-Nal, Ser, Lys, Arg, Nle, Nva, Orn, cyclopropyl Ala, 4-thiazolyl Ala, homoLeu, and absent;
X₃ is selected from the group consisting of Dap, Dab, Thr, Aib, Ala, Cha, Phe, Trp, 1-Nal, Asn, Glu, Lys, Arg, 1Me-Trp, 2-Nal, Gln, Thr, 4-thiazolyl Ala, and absent;
X₄ is selected from the group consisting of Thr, homoArg, Ser, Phe, and absent;
X₅ is selected from the group consisting of Tle, homoArg, Arg, Lys, 4-thiazolyl Ala, Chg, Cha, homoPhe, 4F-Phe, 3F-Phe, 2F-Phe, 4-Pal, 3-Pal, 2-Pal, Cit, Orn, and Phe;
X₆ is selected from the group consisting of Tle, Ile, Phe, Trp, Leu, 1-Nal, Nle, D-Nle, D-Trp, and D-Phe;
X₇ is selected from the group consisting of Thr and Ser;
X₈ is Asp, Cha, Leu, Trp, 1-Nal, Phe, Val, Ala, Abu, Ser, or Thr;
X₉ is Val;
L₁ is a chemical bond or comprises polyethylene glycol in which one or two oxygen atoms are optionally replaced with a nitrogen atom, and CPP is an internalizing peptide.

In an optional embodiment, in the compound represented by formula (G) or formula (I) or the pharmaceutically acceptable salt thereof provided in the present disclosure, X₁ is selected from the group consisting of Gly and absent;
X₂ is selected from the group consisting of Aib, Ala, Ile, Cha, Phe, Trp, 1-Nal, Ser, Lys, Arg, Nle, Nva, Orn, cyclopropyl Ala, 4-thiazolyl Ala, homoLeu, and absent;
X₃ is selected from the group consisting of Dap, Dab, Thr, Aib, Ala, Cha, Phe, Trp, 1-Nal, Asn, Glu, Lys, Arg, 1Me-Trp, 2-Nal, Gln, Thr, 4-thiazolylalanine, and absent;
X₄ is selected from the group consisting of Thr, homoArg, Ser, and absent;
X₅ is selected from the group consisting of Tle, homoArg, and Chg;
X₆ is selected from the group consisting of Tle and Ile;
X₇ is selected from the group consisting of Thr and Ser;
X₈ is Asp;
X₉ is Val.

In some embodiments, in the compound represented by formula (G) or formula (I) or the pharmaceutically acceptable salt thereof provided in the present disclosure, X₇ is Thr.

In some embodiments, in the compound represented by formula (G) or formula (I) or the pharmaceutically acceptable salt thereof provided in the present disclosure, X₆ is Tle.

In some embodiments, in the compound represented by formula (G) or formula (I) or the pharmaceutically acceptable salt thereof provided in the present disclosure, X₅ is Tle.

In some embodiments, in the compound represented by formula (G) or formula (I) or the pharmaceutically acceptable salt thereof provided in the present disclosure, X₆-X₇ is selected from the group consisting of Tle-Thr, Ile-Thr, and Tle-Ser.

In some embodiments, in the compound represented by formula (G) or formula (I) or the pharmaceutically acceptable salt thereof provided in the present disclosure, X₆-X₇ is selected from the group consisting of Tle-Thr and Ile-Thr.

In some embodiments, in the compound represented by formula (G) or formula (I) or the pharmaceutically acceptable salt thereof provided in the present disclosure, X₆-X₇ is Tle-Thr.

In some embodiments, in the compound represented by formula (G) or formula (I) or the pharmaceutically acceptable salt thereof provided in the present disclosure, X₅-X₆ is selected from the group consisting of Tle-Tle, homoArg-Ile, and Chg-Tle.

In some embodiments, in the compound represented by formula (G) or formula (I) or the pharmaceutically acceptable salt thereof provided in the present disclosure, X₅-X₆ is selected from the group consisting of Tle-Tle and Chg-Tle.

In some embodiments, in the compound represented by formula (G) or formula (I) or the pharmaceutically acceptable salt thereof provided in the present disclosure, X₅-X₆ is Tle-Tle.

In some embodiments, in the compound represented by formula (G) or formula (I) or the pharmaceutically acceptable salt thereof provided in the present disclosure, X₅-X₆-X₇ is Tle-Tle-Thr.

In some embodiments, in the compound represented by formula (G) or formula (I) or the pharmaceutically acceptable salt thereof provided in the present disclosure, one or more of X₁, X₂, X₃, and X₄ are optionally absent.

In some embodiments, in the compound represented by formula (G) or formula (I) or the pharmaceutically acceptable salt thereof provided in the present disclosure, X₁ is absent.

In some embodiments, in the compound represented by formula (G) or formula (I) or the pharmaceutically acceptable salt thereof provided in the present disclosure, X₂ and/or X₃ are/is absent.

In some embodiments, in the compound represented by formula (G) or formula (I) or the pharmaceutically acceptable salt thereof provided in the present disclosure, X₁, X₂, X₃, and X₄ are all absent.

In some embodiments, in the compound represented by formula (G) or formula (I) or the pharmaceutically acceptable salt thereof provided in the present disclosure, X₄ is selected from the group consisting of Thr, homoArg, and Ser.

In some embodiments, in the compound represented by formula (G) or formula (I) or the pharmaceutically acceptable salt thereof provided in the present disclosure, X₄ is selected from the group consisting of Thr and Ser.

In some embodiments, in the compound represented by formula (G) or formula (I) or the pharmaceutically acceptable salt thereof provided in the present disclosure, X₄ is Thr.

In some embodiments, in the compound represented by formula (G) or formula (I) or the pharmaceutically acceptable salt thereof provided in the present disclosure, X₃ is selected from the group consisting of Dap and Dab.

In some embodiments, in the compound represented by formula (G) or formula (I) or the pharmaceutically acceptable salt thereof provided in the present disclosure, X₃ is Dab.

In some embodiments, in the compound represented by formula (G) or formula (I) or the pharmaceutically acceptable salt thereof provided in the present disclosure, X₂ is selected from the group consisting of Aib, Ala, and Ile.

In some embodiments, in the compound represented by formula (G) or formula (I) or the pharmaceutically acceptable salt thereof provided in the present disclosure, X₂ is Aib.

In some embodiments, in the compound represented by formula (G) or formula (I) or the pharmaceutically acceptable salt thereof provided in the present disclosure, X₁ is selected from the group consisting of Nle, Gly, and absent.

In some embodiments, in the compound represented by formula (G) or formula (I) or the pharmaceutically acceptable salt thereof provided in the present disclosure, X₁ is Gly.

In some embodiments, in the compound represented by formula (I) or the pharmaceutically acceptable salt thereof provided in the present disclosure, L₁ is a chemical bond.

In some embodiments, in the compound represented by formula (I) or the pharmaceutically acceptable salt thereof provided in the present disclosure, L1 comprises polyethylene glycol in which one or two oxygen atoms are optionally replaced with a nitrogen atom.

In an optional embodiment, L₁ comprises polyethylene glycols, wherein the number of the polyethylene glycols is selected from the group consisting of 0, 1, 2, 3, 4, 5, 6, 7, 8, 9, 10, 11, 12, 13, 14, 15, 16, 17, 18, 19, 20, 21, 22, 23, 24, 25, 26, 27, and 28.

In some embodiments, in the compound represented by formula (I) or the pharmaceutically acceptable salt thereof provided in the present disclosure, L₁ is AEEA.

In some embodiments, in the compound represented by formula (G) or formula (I) or the pharmaceutically acceptable salt thereof provided in the present disclosure, X₁-X₂X₃-X₄-X₅-X₆-X₇-X₈-X₉ is selected from the group consisting of SEQ ID NO. 1 to SEQ ID NO. 8:

| | |
|---|---|
| SEQ ID NO. 1 | G-Aib-Dab-T-Tle-Tle-T-D-V |
| SEQ ID NO. 2 | Tle-Tle-T-D-V |
| SEQ ID NO. 3 | Nle-T-T-Tle-Tle-T-D-V |
| SEQ ID NO. 4 | T-homoArg-homoArg-I-T-D-V |
| SEQ ID NO. 5 | G-Aib-Dab-homoArg-Tle-Tle-T-D-V |
| SEQ ID NO. 6 | G-Aib-Dab-T-Chg-Tle-S-D-V |
| SEQ ID NO. 7 | G-T-Tle-Tle-T-D-V |
| SEQ ID NO. 8 | G-T-Chg-Tle-S-D-V. |

In some embodiments, in the compound represented by formula (I) or the pharmaceutically acceptable salt thereof provided in the present disclosure, the internalizing peptide is from an HIV.

In some embodiments, in the compound represented by formula (I) or the pharmaceutically acceptable salt thereof provided in the present disclosure, the internalizing peptide has a structure as described in WO2022150655A, WO2010072406A, WO2010072405A, or WO2021140485A.

In some embodiments, in the compound represented by formula (I) or the pharmaceutically acceptable salt thereof provided in the present disclosure, the internalizing peptide comprises or is selected from the group consisting of the amino acid sequences set forth in SEQ ID NO: 9 to SEQ ID NO. 11, and an amino acid residue in SEQ ID NO: 9 to SEQ ID NO. 11 is optionally a D-amino acid:

| | |
|---|---|
| SEQ ID NO. 9 | RKKRRQRRR |
| SEQ ID NO. 10 | GRKKRRQRRR |
| SEQ ID NO. 11 | YGRKKRRQRRR. |

In some embodiments, in the compound represented by formula (I) or the pharmaceutically acceptable salt thereof provided in the present disclosure, the internalizing peptide comprises or is selected from the group consisting of the amino acid sequence set forth in SEQ ID NO. 11, and an amino acid residue in SEQ ID NO. 11 is optionally a D-amino acid.

In some embodiments, in the compound represented by formula (I) or the pharmaceutically acceptable salt thereof provided in the present disclosure, the internalizing peptide comprises or is selected from the group consisting of the amino acid sequence set forth in SEQ ID NO. 9, and an amino acid residue in SEQ ID NO. 9 is optionally a D-amino acid.

In an optional embodiment, in the compound represented by formula (I) or the pharmaceutically acceptable salt thereof provided in the present disclosure, 1, 2, 3, 4, 5, 6, 7, 8, 9, 10, or 11 of the amino acid residues of the internalizing peptide are D-amino acids.

In an optional embodiment, in the compound represented by formula (I) or the pharmaceutically acceptable salt thereof provided in the present disclosure, 4, 5, 6, 7, 8, 9, 10, or 11 of the amino acid residues of the internalizing peptide are D-amino acids.

In an optional embodiment, in the compound represented by formula (I) or the pharmaceutically acceptable salt thereof provided in the present disclosure, 9, 10, or 11 of the amino acid residues of the internalizing peptide are D-amino acids.

In an optional embodiment, in the compound represented by formula (I) or the pharmaceutically acceptable salt thereof provided in the present disclosure, an amino acid residue R of the internalizing peptide is a D-amino acid.

In an optional embodiment, in the compound represented by formula (I) or the pharmaceutically acceptable salt thereof provided in the present disclosure, 1, 2, 3, 4, 5, or 6 of the amino acid residues R are D-amino acids.

In an optional embodiment, in the compound represented by formula (I) or the pharmaceutically acceptable salt thereof provided in the present disclosure, 3, 4, 5, or 6 of the amino acid residues R are D-amino acids.

In an optional embodiment, in the compound represented by formula (I) or the pharmaceutically acceptable salt thereof provided in the present disclosure, 5 or 6 of the amino acid residues R are D-amino acids.

In an optional embodiment, in the compound represented by formula (I) or the pharmaceutically acceptable salt thereof provided in the present disclosure, the first amino acid residue R from the C-terminus of the internalizing peptide is a D-amino acid, and the amino acid residue that is optionally 0, 1, 2, 3, 4, 5, 6, 7, 8, 9, or 10 amino acids apart from the first amino acid residue R is a D-amino acid.

In an optional embodiment, in the compound represented by formula (I) or the pharmaceutically acceptable salt thereof provided in the present disclosure, the first amino acid residue R from the C-terminus of the internalizing peptide is a D-amino acid, and the amino acid residue that is optionally 0, 1, 2, 3, 4, 5, 6, 7, or 8 amino acids apart from the first amino acid residue R is a D-amino acid.

In an optional embodiment, in the compound represented by formula (I) or the pharmaceutically acceptable salt thereof provided in the present disclosure, the first amino acid residue R from the C-terminus of the internalizing peptide is a D-amino acid, and the amino acid residue that is optionally 0, 1, 2, or 3 amino acids apart from the first amino acid residue R is a D-amino acid.

In an optional embodiment, in the compound represented by formula (I) or the pharmaceutically acceptable salt thereof provided in the present disclosure, the internalizing peptide comprises or is selected from the group consisting of the amino acid sequences set forth in SEQ ID NO: 12 and SEQ ID NO: 13:

| | |
|---|---|
| SEQ ID NO. 12 | y-G-r-k-k-r-r-q-r-r-r |
| SEQ ID NO. 13 | r-K-K-R-r-Q-R-R-r. |

| | |
|---|---|
| Note: The single lower-case letters represent amino acids in D-configuration. | |

In an optional embodiment, the compound represented by formula (I) or the pharmaceutically acceptable salt thereof provided in the present disclosure is selected from the group consisting of SEQ ID NO. 14 to SEQ ID NO. 22:

| | |
|---|---|
| SEQ ID NO. 14 | y-G-r-k-k-r-r-q-r-r-r-G-Aib-Dab-T-Tle-Tle-T-D-V |
| SEQ ID NO. 15 | y-G-r-k-k-r-r-q-r-r-r-AEEA-Tle-Tle-T-D-V |
| SEQ ID NO. 16 | y-G-r-k-k-r-r-q-r-r-r-Nle-T-T-Tle-Tle-T-D-V |
| SEQ ID NO. 17 | y-G-r-k-k-r-r-q-r-r-r-T-homoArg-homoArg-I-T-D-V |
| SEQ ID NO. 18 | y-G-r-k-k-r-r-q-r-r-r-G-Aib-Dab-homoArg-Tle-Tle-T-D-V |
| SEQ ID NO. 19 | y-G-r-k-k-r-r-q-r-r-r-G-Aib-Dab-T-Chg-Tle-S-D-V |
| SEQ ID NO. 20 | y-G-r-k-k-r-r-q-r-r-r-G-T-Tle-Tle-T-D-V |
| SEQ ID NO. 21 | y-G-r-k-k-r-r-q-r-r-r-G-T-Chg-Tle-S-D-V |
| SEQ ID NO. 22 | r-K-K-R-r-Q-R-R-r-G-Aib-Dab-T-Tle-Tle-T-D-V. |

| | |
|---|---|
| Note: The single lower-case letters represent amino acids in D-configuration. | |

In another aspect, the present disclosure provides an active peptide or a pharmaceutically acceptable salt thereof, wherein the active peptide comprises or consists of formula (G):

X₁-X₂-X₃-X₄-X₅-X₆-X₇-X₈-X₉ (G),

wherein X₁, X₂, X₃, X₄, X₅, X₆, X₇, X₈, and X₉ are each as defined in the compound represented by formula (I).

In some embodiments, the number of amino acid residues of the active peptide is selected from the group consisting of 5, 6, 7, 8, 9, 10, 11, 12, 13, 14, and 15.

In some embodiments, the active peptide comprises or is selected from the group consisting of the amino acid sequences set forth in SEQ ID NO. 1 to SEQ ID NO. 8.

In another aspect, the present disclosure provides a peptide comprising an active peptide and an internalizing peptide, wherein the active peptide comprises or is selected from the group consisting of the amino acid sequences set forth in SEQ ID NO. 1 to SEQ ID NO. 8, and the internalizing peptide is as defined in the present disclosure. In some embodiments, the internalizing peptide comprises or is selected from the group consisting of the amino acid sequences set forth in SEQ ID NO: 12 and SEQ ID NO. 13.

In some embodiments, the peptide comprises or is selected from the group consisting of the amino acid sequences set forth in SEQ ID NO. 14 to SEQ ID NO. 22.

In another aspect, the present disclosure provides an active peptide comprising or selected from the group consisting of SEQ ID NO. 23 to SEQ ID NO. 39 as follows:

| | |
|---|---|
| SEQ ID NO. 23 | S-S-Tle-I-S-T-V |
| SEQ ID NO. 24 | Tle-I-S-T-V |
| SEQ ID NO. 25 | Tle-Tle-T-T-V |
| SEQ ID NO. 26 | G-S-T-E-S-W-V |
| SEQ ID NO. 27 | G-S-Orn-E-S-W-V |
| SEQ ID NO. 28 | S-r-r-I-S-T-V |
| SEQ ID NO. 29 | S-homoArg-homoArg-I-S-T-V |
| SEQ ID NO. 30 | G-S-homoArg-E-S-W-V |
| SEQ ID NO. 31 | G-S-Orn-Tle-S-W-V |
| SEQ ID NO. 32 | G-T-Orn-E-S-W-V |
| SEQ ID NO. 33 | G-T-Orn-E-S-Abu-V |
| SEQ ID NO. 34 | G-T-Tle-E-T-W-V |
| SEQ ID NO. 35 | G-k-Tle-E-T-W-V |
| SEQ ID NO. 36 | G-T-Dab-E-T-W-V |
| SEQ ID NO. 37 | A-R-R-E-T-A-V |
| SEQ ID NO. 38 | Tle-E-T-T-V |
| SEQ ID NO. 39 | G-Aib-Dab-T-Tle-E-T-T-V. |

In some embodiments, the active peptide selected from the group consisting of SEQ ID NO. 23 to SEQ ID NO. 39 provided in the present disclosure is linked to an internalizing peptide.

In an optional embodiment, the internalizing peptide has a structure as described in WO2022150655A, WO2010072406A, WO2010072405A, or WO2021140485A.

In an optional embodiment, the internalizing peptide comprises or is selected from the group consisting of the amino acid sequence set forth in any one of SEQ ID NO: 9 to SEQ ID NO. 11, and an amino acid residue in SEQ ID NO: 9 to SEQ ID NO. 11 is optionally a D-amino acid:

| | |
|---|---|
| SEQ ID NO. 9 | RKKRRQRRR |
| SEQ ID NO. 10 | GRKKRRQRRR |
| SEQ ID NO. 11 | YGRKKRRQRRR. |

In an optional embodiment, the active peptide represented by SEQ ID NO. 23 to SEQ ID NO. 39 is optionally linked to the internalizing peptide by L₂, wherein L₂ comprises polyethylene glycol in which one or two oxygen atoms are optionally replaced with a nitrogen atom.

In an optional embodiment, L₂ comprises polyethylene glycols, wherein the number of the polyethylene glycols is selected from the group consisting of 0, 1, 2, 3, 4, 5, 6, 7, 8, 9, 10, 11, 12, 13, 14, 15, 16, 17, 18, 19, 20, 21, 22, 23, 24, 25, 26, 27, and 28.

In some embodiments, L₂ is AEEA.

In an optional embodiment, the active peptide represented by SEQ ID NO. 23 to SEQ ID NO. 39 forms, with the internalizing peptide, the peptides represented by SEQ ID NO. 40 to SEQ ID NO. 61 as follows:

| | |
|---|---|
| SEQ ID NO. 40 | y-G-r-k-k-r-r-q-r-r-r-S-S-Tle-I-S-T-V |
| SEQ ID NO. 41 | y-G-r-k-k-r-r-q-r-r-r-AEEA-Tle-I-S-T-V |
| SEQ ID NO. 42 | y-G-r-k-k-r-r-q-r-r-r-AEEA-Tle-Tle-T-T-V |
| SEQ ID NO. 43 | y-G-r-k-k-r-r-q-r-r-r-G-S-T-E-S-W-V |
| SEQ ID NO. 44 | y-G-r-k-k-r-r-q-r-r-r-G-S-Orn-E-S-W-V |
| SEQ ID NO. 45 | y-G-r-k-k-r-r-q-r-r-r-S-r-r-I-S-T-V |
| SEQ ID NO. 46 | y-G-r-k-k-r-r-q-r-r-r-S-homoArg-homoArg-I-S-T-V |
| SEQ ID NO. 47 | y-G-r-k-k-r-r-q-r-r-r-G-S-homoArg-E-S-W-V |
| SEQ ID NO. 48 | y-G-r-k-k-r-r-q-r-r-r-G-S-Orn-Tle-S-W-V |
| SEQ ID NO. 49 | y-G-r-k-k-r-r-q-r-r-r-G-T-Orn-E-S-W-V |
| SEQ ID NO. 50 | y-G-r-k-k-r-r-q-r-r-r-G-T-Orn-E-S-Abu-V |
| SEQ ID NO. 51 | y-G-r-k-k-r-r-q-r-r-r-G-T-Tle-E-T-W-V |
| SEQ ID NO. 52 | y-G-r-k-k-r-r-q-r-r-r-G-k-Tle-E-T-W-V |
| SEQ ID NO. 53 | y-G-r-k-k-r-r-q-r-r-r-G-T-Dab-E-T-W-V |
| SEQ ID NO. 54 | y-G-r-k-k-r-r-q-r-r-r-A-R-R-E-T-A-V |
| SEQ ID NO. 55 | y-G-r-k-k-r-r-q-r-r-r-AEEA-Tle-E-T-T-V |
| SEQ ID NO. 56 | y-G-r-k-k-r-r-q-r-r-r-G-Aib-Dab-T-Tle-E-T-T-V |
| SEQ ID NO. 57 | r-r-r-q-r-r-k-k-r-G-Aib-Dab-T-Tle-Tle-T-D-V |
| SEQ ID NO. 58 | Ac-y-G-r-K-k-R-r-Q-r-R-r-G-Aib-Dab-T-Tle-Tle-T-D-V |
| SEQ ID NO. 59 | r-r-r-q-r-r-k-k-r-G-Aib-Dab-T-Chg-Tle-S-D-V |
| SEQ ID NO. 60 | Ac-y-G-r-K-k-R-r-Q-r-R-r-G-Aib-Dab-T-Chg-Tle-S-D-V |
| SEQ ID NO. 61 | r-K-K-R-r-Q-R-R-r-G-Aib-Dab-T-Chg-Tle-S-D-V. |

| | |
|---|---|
| Note: The single lower-case letters represent amino acids in D-configuration. | |

In some embodiments, the peptide of the present disclosure comprises or is selected from the group consisting of the amino acid sequences set forth in SEQ ID NO. 40 to SEQ ID NO. 61.

The active peptide of the present disclosure (without the internalizing peptide) is 3-25 amino acids, 4-15 amino acids, 4-10 amino acids, e.g., 9 amino acids, e.g., 3, 4, 5, 6, 7, 8, or 9 amino acids, in length.

The compound represented by formula (G) or the pharmaceutically acceptable salt thereof, the compound represented by formula (I) or the pharmaceutically acceptable salt thereof, and the active peptide or the peptide or the pharmaceutically acceptable salt thereof provided in the present disclosure are active peptides capable of binding to the PDZ1 or PDZ2 domain of PSD-95.

The compound represented by formula (G) or the pharmaceutically acceptable salt thereof, the compound represented by formula (I) or the pharmaceutically acceptable salt thereof, and the active peptide or the peptide or the pharmaceutically acceptable salt thereof provided in the present disclosure can be used as inhibitors of PSD-95-mediated protein-protein interactions.

In another aspect, the present disclosure further provides a pharmaceutical composition comprising a therapeutically effective amount of the aforementioned compound or pharmaceutically acceptable salt thereof, and/or the aforementioned active peptide or peptide or pharmaceutically acceptable salt thereof, and a pharmaceutically acceptable carrier and/or excipient.

In some embodiments, a unit dose of the pharmaceutical composition is 0.001 mg-1000 mg.

In certain embodiments, the pharmaceutical composition comprises 0.01-99.99% of the aforementioned compound or pharmaceutically acceptable salt thereof or the aforementioned active peptide or peptide or pharmaceutically acceptable salt thereof on the basis of the total weight of the composition. In certain embodiments, the pharmaceutical composition comprises 0.1-99.9% of the aforementioned compound or pharmaceutically acceptable salt thereof or the aforementioned active peptide or peptide or pharmaceutically acceptable salt thereof. In certain embodiments, the pharmaceutical composition comprises 0.5%-99.5% of the aforementioned compound or pharmaceutically acceptable salt thereof or the aforementioned active peptide or peptide or pharmaceutically acceptable salt thereof. In certain embodiments, the pharmaceutical composition comprises 1%-99% of the aforementioned compound or pharmaceutically acceptable salt thereof or the aforementioned active peptide or peptide or pharmaceutically acceptable salt thereof. In certain embodiments, the pharmaceutical composition comprises 2%-98% of the aforementioned compound or pharmaceutically acceptable salt thereof or the aforementioned active peptide or peptide or pharmaceutically acceptable salt thereof.

In certain embodiments, the pharmaceutical composition comprises 0.01%-99.99% of the pharmaceutically acceptable excipient based on the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

The present disclosure provides the aforementioned compound represented by formula (G) or pharmaceutically acceptable salt thereof, the compound represented by formula (I) or the pharmaceutically acceptable salt thereof, or the aforementioned active peptide or peptide or pharmaceutically acceptable salt thereof for use as a medicament.

The present disclosure provides the aforementioned compound represented by formula (G) or pharmaceutically acceptable salt thereof, the compound represented by formula (I) or the pharmaceutically acceptable salt thereof, the aforementioned active peptide or peptide or pharmaceutically acceptable salt thereof, or the aforementioned pharmaceutical composition, which can improve neurological function in the brain and reduce cerebral infarct size.

The present disclosure provides the aforementioned compound represented by formula (G) or pharmaceutically acceptable salt thereof, the compound represented by formula (I) or the pharmaceutically acceptable salt thereof, the aforementioned active peptide or peptide or pharmaceutically acceptable salt thereof, or the aforementioned pharmaceutical composition, which can reduce the ability to release histamine.

The present disclosure provides the aforementioned compound represented by formula (G) or pharmaceutically acceptable salt thereof, the compound represented by formula (I) or the pharmaceutically acceptable salt thereof, the aforementioned active peptide or peptide or pharmaceutically acceptable salt thereof, or the aforementioned pharmaceutical composition, which can increase plasma exposure.

The present disclosure provides the aforementioned compound represented by formula (G) or pharmaceutically acceptable salt thereof, the compound represented by formula (I) or the pharmaceutically acceptable salt thereof, the aforementioned active peptide or peptide or pharmaceutically acceptable salt thereof, or the aforementioned pharmaceutical composition, which can extend T_{1/2}.

The present disclosure provides the compound represented by formula (G) or the pharmaceutically acceptable salt thereof, the compound represented by formula (I) or the pharmaceutically acceptable salt thereof, the aforementioned active peptide or peptide or pharmaceutically acceptable salt thereof, or the aforementioned pharmaceutical composition, which has high plasma stability.

In another aspect, the present disclosure provides use of the aforementioned compound represented by formula (G) or pharmaceutically acceptable salt thereof, the compound represented by formula (I) or the pharmaceutically acceptable salt thereof, the aforementioned active peptide or peptide or pharmaceutically acceptable salt thereof, or the pharmaceutical composition in the manufacture of a medicament for treating and/or preventing strokes, cerebral ischemia, traumatic injury to the central nervous system, reperfusion injury, subarachnoid hemorrhage, concussion, pain, anxiety, epilepsy, or neurodegenerative diseases (Alzheimer's disease or Parkinson's disease) or diseases involving the risks described above.

In another aspect, the present disclosure provides a method for treating and/or preventing strokes, cerebral ischemia, traumatic injury to the central nervous system, reperfusion injury, subarachnoid hemorrhage, concussion, pain, anxiety, epilepsy, or neurodegenerative diseases (Alzheimer's disease or Parkinson's disease) or diseases involving the risks described above, the method comprising administering to a patient a therapeutically effective amount or a prophylactically effective amount of the aforementioned compound represented by formula (G) or pharmaceutically acceptable salt thereof, the compound represented by formula (I) or the pharmaceutically acceptable salt thereof, the aforementioned active peptide or peptide or pharmaceutically acceptable salt thereof, or the aforementioned pharmaceutical composition.

In another aspect, the present disclosure provides use of the aforementioned compound represented by formula (G) or pharmaceutically acceptable salt thereof, the compound represented by formula (I) or the pharmaceutically acceptable salt thereof, the aforementioned active peptide or peptide or pharmaceutically acceptable salt thereof, or the aforementioned pharmaceutical composition in combination with a thrombolytic drug in the manufacture of a medicament for treating and/or preventing acute ischemic stroke (AIS). In another aspect, the present disclosure provides a method for treating and/or preventing acute ischemic stroke (AIS), the method comprising administering to a patient a therapeutically effective amount or a prophylactically effective amount of the aforementioned compound represented by formula (G) or pharmaceutically acceptable salt thereof, the compound represented by formula (I) or the pharmaceutically acceptable salt thereof, the aforementioned active peptide or peptide or pharmaceutically acceptable salt thereof, or the aforementioned pharmaceutical composition and a thrombolytic drug.

In another aspect, the present disclosure provides the aforementioned compound represented by formula (G) or pharmaceutically acceptable salt thereof, the compound represented by formula (I) or the pharmaceutically acceptable salt thereof, the aforementioned active peptide or peptide or pharmaceutically acceptable salt thereof, or the aforementioned pharmaceutical composition for use in treating and/or preventing acute ischemic stroke (AIS), wherein the aforementioned compound represented by formula (G) or pharmaceutically acceptable salt thereof, the compound represented by formula (I) or the pharmaceutically acceptable salt thereof, the aforementioned active peptide or peptide or pharmaceutically acceptable salt thereof, or the aforementioned pharmaceutical composition is used in combination with a thrombolytic drug. The two different active ingredients are in the same container or different containers.

In another aspect, the present disclosure provides a thrombolytic drug for treating and/or preventing acute ischemic stroke (AIS), wherein the thrombolytic drug is used in combination with the aforementioned compound represented by formula (G) or pharmaceutically acceptable salt thereof, the compound represented by formula (I) or the pharmaceutically acceptable salt thereof, the aforementioned active peptide or peptide or pharmaceutically acceptable salt thereof, or the aforementioned pharmaceutical composition. The two different active ingredients are in the same container or different containers.

The thrombolytic drug in the present disclosure is selected from the group consisting of urokinase, streptokinase, anistreplase, staphylokinase, recombinant staphylokinase, prourokinase, a *Desmodus rotundus* salivary plasminogen activator, lanoteplase, pamiteplase, monteplase, alteplase, reteplase, tenecteplase, and the like.

In an optional embodiment, the thrombolytic drug of the present disclosure is alteplase (rt-PA).

In another aspect, the present disclosure provides a method for treating an injury to the central nervous system caused by ischemia, the method comprising administering to a patient with ischemia or at risk of ischemia a therapeutically effective amount of the aforementioned compound represented by formula (G) or pharmaceutically acceptable salt thereof, the compound represented by formula (I) or the pharmaceutically acceptable salt thereof, the aforementioned active peptide or peptide or pharmaceutically acceptable salt thereof, or the aforementioned composition, and administering reperfusion therapy to the patient. For explanations of "ischemia", "reperfusion", and "reperfusion therapy" in the present disclosure, reference can be made to WO2012176172A.

In the polypeptide compound sequences provided in the present disclosure, the single lower-case letters represent amino acids in D-configuration. Unless otherwise specified, single capital letters represent amino acids in L-configuration (wherein Gly and Aib have no steric configurations), e.g., RKKRRQRRR (SEQ ID NO: 9). When it is specified that an amino acid residue in SEQ ID NO: 9 is optionally a D-amino acid, then the amino acid is optionally an amino acid in D-configuration.

The compounds and derivatives thereof provided in the present disclosure are synthesized by solid-phase synthesis using Fmoc-Val-Wang resin as the support. During synthesis, the α-amino groups of the amino acid derivatives used are protected by the Fmoc group (fluorenylmethoxycarbonyl), and the side chains of the amino acids are protected using protecting groups according to their functional groups as follows: the side-chain amino group of glutamine (L/D) is protected by Trt (trityl); the side-chain guanidino groups of homoarginine (homoArg) and arginine (L/D) are protected by Pbf (2,2,4,6,7-pentamethyldihydrobenzofuran-5-sulfonyl); the side-chain indolyl group of tryptophan, the side-chain amino group of lysine (L/D), and the side-chain amino groups of Dab and Orn are protected by Boc (tert-butoxycarbonyl); the side-chain hydroxy group of threonine, the side-chain phenol group of tyrosine (L/D), the side-chain hydroxy group of serine, the side-chain carboxyl group of aspartic acid, and the side-chain carboxyl group of glutamic acid are protected by t-Bu (tert-butyl). During synthesis, Fmoc-Val-Wang resin is first fully swollen in N,N-dimethylformamide (DMF), the Fmoc protecting group on the α-amino group is removed using a 20% solution of 4-methylpiperidine in DMF, and the carboxyl group of the C-terminal amino acid residue is then condensed in the form of an amide bond onto the insoluble polymeric resin. The Fmoc protecting group on the α-amino group is then removed using a 20% solution of 4-methylpiperidine in DMF. The solid-phase support is then reacted by condensation with the next amino acid derivative in the sequence in excess to form an amide bond, thereby extending the peptide chain. The procedure of washing → deprotection → washing → the next round of amino acid condensation→ washing is repeated until the desired polypeptide chain length is achieved. Finally, the resin is reacted with a mixture of trifluoroacetic acid, water, and triisopropylsilane (90:5:5, v:v:v) to cleave the polypeptide from the solid-phase support, and a solid is then precipitated using cold methyl tert-butyl ether. After centrifugation, the supernatant is removed to obtain a crude polypeptide product, and the crude product is dried overnight. The crude polypeptide solid is dissolved in deionized water and purified and separated by C-18 reversed-phase preparative chromatography to obtain the purified polypeptide and derivatives thereof.

### Detailed Description of the Invention

"Internalizing peptide" refers to a well-known class of relatively short peptides that enable many cellular or viral proteins to cross cell membranes. Internalizing peptides, also known as cell membrane transduction peptides or cell-penetrating peptides, have, for example, 5-30 amino acids. These peptides often have a cationic charge derived from arginine and/or lysine residues (usually relative to proteins) that are considered to facilitate their membrane crossing. Some such polypeptides have at least 5, 6, 7, or 8 arginine and/or lysine residues.

"Natural amino acids" refers to 20 conventional amino acids, i.e., alanine (A), cysteine (C), aspartic acid (D), glutamic acid (E), phenylalanine (F), glycine (G), histidine (H), isoleucine (I), lysine (K), leucine (L), methionine (M), asparagine (N), proline (P), glutamine (Q), arginine (R), serine (S), threonine (T), valine (V), tryptophan (W), and tyrosine (Y).

"Non-natural amino acids" refers to amino acids that are not naturally encoded or found in the genetic code of any organism. They may be completely synthetic compounds. Examples of non-natural amino acids include, but are not limited to, hydroxyproline, γ-carboxyglutamic acid, O-phosphoserine, azetidinecarboxylic acid, 2-aminoadipic acid, 3-aminoadipic acid, β-alanine, aminopropionic acid, 2-aminobutyric acid, 4-aminobutyric acid, 6-aminohexanoic acid, 2-aminoheptanoic acid, 2-aminoisobutyric acid (Aib), 3-aminoisobutyric acid, 2-aminopimelic acid, tert-butylglycine, 2,4-diaminoisobutyric acid (Dap), tert-leucine (Tle), desmosine, 2,2'-diaminopimelic acid, 2,3-diaminopropionic acid (Dab), N-ethylglycine, N-methylglycine, N-ethylasparagine, homoproline, hydroxylysine, allo-hydroxylysine, 3-hydroxyproline, 4-hydroxyproline, isodesmosine, allo-isoleucine, N-methylalanine, N-methylglycine, N-methylisoleucine, N-methylpentylglycine, N-methylvaline, naphthalanine, norvaline, norleucine, ornithine (Orn), D-ornithine, D-arginine, homoarginine (homoArg), D-tyrosine, D-lysine, D-glutamine, p-aminophenylalanine, pentylglycine, pipecolic acid, and thioproline. Furthermore, non-natural amino acids also include those in which the C-terminal carboxyl groups, N-terminal amino groups, and/or side-chain functional groups of natural amino acids or non-natural amino acids are chemically modified.

The abbreviations for some amino acids in the present disclosure and their corresponding structural abbreviations are as follows:

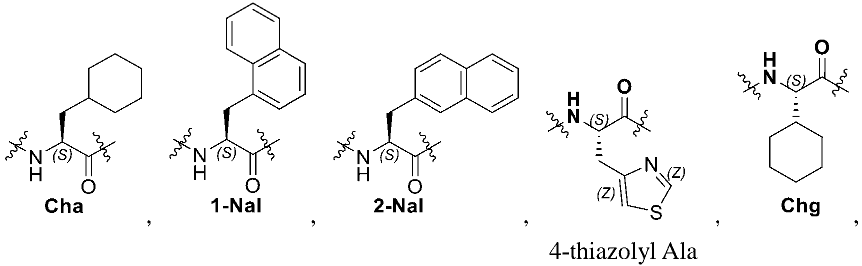

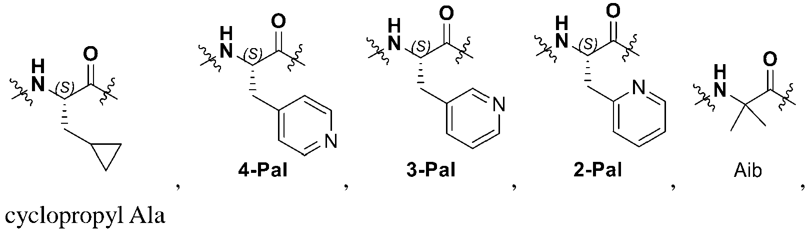

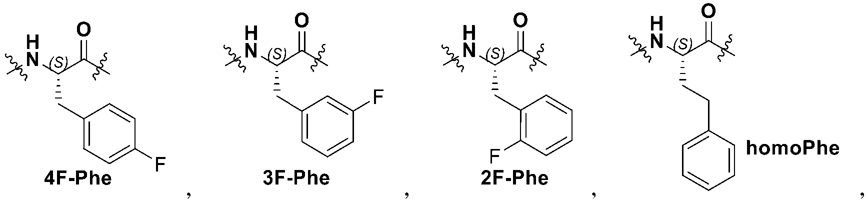

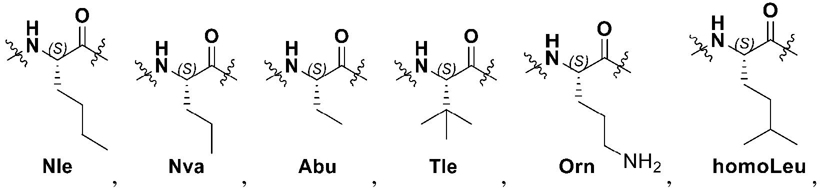

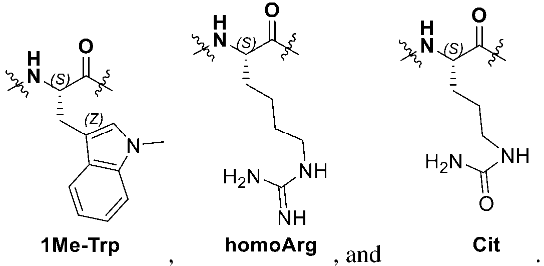

The term "AEEA" is structurally shown as follows:

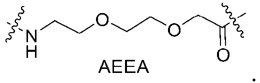

"Optionally" or "optional" means that the event or circumstance subsequently described may, but does not necessarily, occur, and this description includes instances where the event or circumstance occurs or does not occur. For example, "C1-6 alkyl optionally substituted with halogen or cyano" means that the halogen or cyano may, but does not necessarily, exist, and this description includes the instance where the alkyl is substituted with halogen or cyano and the instance where the alkyl is not substituted with halogen or cyano.

In the chemical structures of the compounds of the present disclosure, unless otherwise specified, the bond " 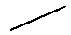 " indicates an unspecified configuration; that is, if chiral isomers exist in the chemical structures, the bond " 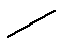 " may be " 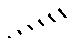 " or " 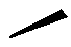 ", or may include both " 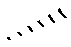 " and " 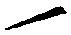 " configurations simultaneously. Although all of the above structural formulas are drawn as certain isomeric forms for the sake of simplicity, the present disclosure may include all isomers, such as tautomers, rotamers, geometric isomers, diastereomers, racemates, and enantiomers.

The term "subject" or "patient" includes humans and veterinary animals, e.g., mammals, as well as laboratory animal models, e.g., mice or rats for preclinical studies.

As used in the present disclosure, "Ac-y" means that the amino group of amino acid y is acetylated.

### BRIEF DESCRIPTION OF THE DRAWINGS

FIG. 1A. A hemolysis risk test in rat whole blood.
FIG. 1B. A hemolysis risk test in human whole blood.
FIG. 1C. A hemolysis risk test in rabbit whole blood.
FIG. 2A. A stability test of a polypeptide compound in human plasma.
FIG. 2B. A stability test of a polypeptide compound in rat plasma.
FIG. 2C. A stability test of a polypeptide compound in human plasma.
FIG. 2D. A stability test of a polypeptide compound in rat plasma.
FIG. 3. Pharmacokinetic results of polypeptide compounds in rats.
FIG. 4. Pharmacokinetic results of different doses of polypeptide compound 14.
FIG. 5A. Rat nephrotoxicity results of a polypeptide compound - blood urea nitrogen levels (ns indicates no statistical difference, * indicates P < 0.05, and ** indicates P < 0.01).
FIG. 5B. Rat nephrotoxicity results of a polypeptide compound - serum creatinine levels (ns indicates no statistical difference).
FIG. 6. Pharmacokinetic results of a polypeptide compound in beagle dogs.
FIG. 7. The ability of a polypeptide compound to induce histamine release in beagle dogs.
FIG. 8A. Pharmacodynamic results of polypeptide compound 14 in a rat tMCAO model - cerebral infarct size (** indicates P < 0.01, and *** indicates P < 0.001).
FIG. 8B. Pharmacodynamic results of polypeptide compound 14 in a rat tMCAO model - neurological function impairment scores (* indicates P < 0.05, and ** indicates P < 0.01).
FIG. 8C. Pharmacodynamic results of polypeptide compound 22 in a rat tMCAO model - cerebral infarct size (* indicates P < 0.05, and ** indicates P < 0.01).
FIG. 8D. Pharmacodynamic results of polypeptide compound 22 in a rat tMCAO model - neurological function impairment scores (* indicates P < 0.05, and ** indicates P < 0.01).

### DETAILED DESCRIPTION

To illustrate the present disclosure in greater detail, the specification provides the following specific embodiments. However, the solutions of the present disclosure are not limited to these embodiments.

### 1. Reagents

**Table 1-1**

| No. | Reagent | Source |
|---|---|---|
| 1 | Fmoc-Val-Wang resin (loading: 0.349 mmol/g) | GL Biochem |
| 2 | O-(7-Azabenzotriazol-1-yl)-N,N,N',N'-tetramethyluronium hexafluorophosphate (HATU) | GL Biochem |
| 3 | 4-Methylpiperidine | TCI |
| 4 | 4-Methylmorpholine | TCI |
| 5 | Methyl tert-butyl ether | TCI |
| 6 | Trifluoroacetic acid (TFA) | TCI |
| 7 | Triisopropylsilane | TCI |
| 8 | N,N-Dimethylformamide (DMF) | Tianjin Fuchen |
| 9 | Dichloromethane (DCM) | Tianjin Fuchen |
| 10 | Acetonitrile | Shanghai CINC |
| 11 | Formic acid (FA) | Thermo |
| 12 | Ammonium bicarbonate | Sigma |
| 13 | Fmoc-Gly-OH | GL Biochem |
| 14 | Fmoc-Asp(OtBu)-OH | GL Biochem |
| 15 | Fmoc-Thr(tBu)-OH | GL Biochem |
| 16 | Fmoc-Ser(tBu)-OH | GL Biochem |
| 17 | Fmoc-Tle-OH | GL Biochem |
| 18 | Fmoc-Dab(Boc)-OH | GL Biochem |
| 19 | Fmoc-Aib-OH | GL Biochem |
| 20 | Fmoc-Nle-OH | GL Biochem |
| 21 | Fmoc-homoArg(Pbf)-OH | GL Biochem |
| 22 | Fmoc-AEEA-OH | GL Biochem |
| 23 | Fmoc-Chg-OH | GL Biochem |
| 24 | Fmoc-D-Gln(Trt)-OH | GL Biochem |
| 25 | Fmoc-D-Lys(Boc)-OH | GL Biochem |
| 26 | Fmoc-D-Arg(Pbf)-OH | GL Biochem |
| 27 | Fmoc-D-Tyr(tBu)-OH | GL Biochem |
| 28 | Fmoc-Gln(Trt)-OH | GL Biochem |
| 29 | Fmoc-Lys(Boc)-OH | GL Biochem |
| 30 | Fmoc-Arg(Pbf)-OH | GL Biochem |
| 31 | Fmoc-Ile-OH | GL Biochem |

### 2. Instruments

**Table 1-2**

| No. | Instrument | Source |
|---|---|---|
| 1 | H-CLASS analytical ultra-high performance liquid chromatograph | WATERS |
| 2 | 6530 liquid chromatography/mass spectrometry system | Agilent |
| 3 | Labconco multifunctional freeze dryer | Thermo-Fisher Scientific |
| 4 | Prep150 preparative high performance liquid chromatograph | WATERS |
| 5 | Multichannel high-speed centrifuge | Sigma |

### 3. Specific experimental protocols

### 3.1. Chemical synthesis of polypeptide compound 14

y-G-r-k-k-r-r-q-r-r-r-G-Aib-Dab-T-Tle-Tle-T-D-V (SEQ ID NO. 14)

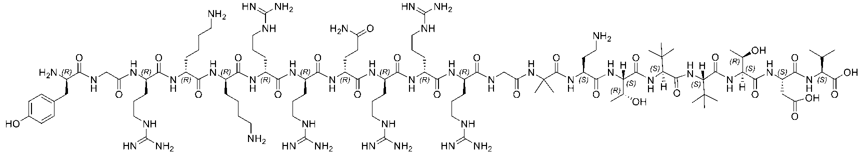

### 3.1.1. Resin swelling and Fmoc protecting group removal

The solid-phase synthesis support Fmoc-Val-Wang resin (286 mg, 0.1 mmol, loading: 0.349

mmol/g) was weighed out and placed in a disposable polypropylene polypeptide synthesis solid-phase reaction tube, and DMF (10 mL) was added. The resin was swollen for 10 min, and DMF was removed under vacuum. DMF (5 mL) was added to wash the resin, and the washing step was repeated twice. Then the solvent was removed under vacuum. 4-Methylpiperidine/DMF (20% v/v, 5 mL) was added to the above resin, and the mixture was shaken at room temperature for 8 min before the resin was drained. 4-Methylpiperidine/DMF (20% v/v, 5 mL) was added again, and the mixture was shaken at room temperature for 8 min. Then the solvent was removed under vacuum. After the deprotection was complete, the resin was washed four times with DMF (5 mL).

### 3.1.2. Coupling of peptide chain sequence

Polypeptide compound 14 was synthesized from the carboxyl-terminus to the amino-terminus according to its peptide chain sequence. First, Fmoc-Asp(OtBu)-OH (1 mmol) was weighed out and dissolved in DMF to prepare a 0.34 M solution. O-(7-Azabenzotriazol-1-yl)-N,N,N',N'-tetramethyluronium hexafluorophosphate (HATU) (1 mmol) was weighed out and dissolved in DMF to prepare a 0.34 M solution. 4-Methylmorpholine (NMM, 2 mmol) was measured out and dissolved in DMF to prepare a 1 M solution. 3 mL of the Fmoc-Asp(OtBu)-OH solution, 3 mL of the HATU solution, and 1.5 mL of the 4-methylmorpholine solution were mixed and added to the resin obtained in step 3.1.1, and the mixture was shaken at room temperature for 35 min. After the reaction was complete, the resin was washed three times with DMF.

4-Methylpiperidine/DMF (20% v/v, 5 mL) was added to the above resin to remove the Fmoc protecting group from the N-terminus of the amino acid. The mixture was shaken at room temperature for 8 min, and the solvent was removed under vacuum. 4-Methylpiperidine/DMF (20% v/v, 5 mL) was added again, and the mixture was shaken at room temperature for 8 min. Then the solvent was removed under vacuum. After the reaction was complete, the resin was washed three times with DMF (5 mL). The above amino acid derivative condensation process was repeated to sequentially condense: Fmoc-Thr(tBu)-OH, Fmoc-Tle-OH, Fmoc-Tle-OH, Fmoc-Thr(tBu)-OH, Fmoc-Dab(Boc)-OH, Fmoc-Aib-OH, Fmoc-Gly-OH, Fmoc-D-Arg(Pbf)-OH, Fmoc-D-Arg(Pbf)-OH, Fmoc-D-Arg(Pbf)-OH, Fmoc-D-Gln(Trt)-OH, Fmoc-D-Arg(Pbf)-OH, Fmoc-D-Arg(Pbf)-OH, Fmoc-D-Lys(Boc)-OH, Fmoc-D-Lys(Boc)-OH, Fmoc-D-Arg(Pbf)-OH, Fmoc-Gly-OH, and Fmoc-D-Tyr(tBu)-OH. Finally, the complete resin-peptide containing polypeptide compound 14 was obtained.

### 3.1.3. Cleavage of resin-peptide

The resin-peptide containing polypeptide compound 14 obtained from the above step was sequentially washed with DMF and DCM three times and then dried *in vacuo,* and 8 mL of a freshly prepared cleavage solution (trifluoroacetic acid:triisopropylsilane:water = 90:5:5, v:v:v) was then added. The mixture was shaken at room temperature for 6 h. After the reaction was complete, the mixture was filtered, and the resin was washed twice with trifluoroacetic acid. After the filtrates were combined, a large amount of cold methyl tert-butyl ether was added to precipitate a solid. After centrifugation, the supernatant was discarded, and a crude polypeptide product was obtained. Then the product was dried overnight.

### 3.1.4. Reversed-phase liquid chromatography purification of crude peptide product

The crude peptide product was purified twice: first with a TFA system, and then with a 25 mM ammonium bicarbonate (pH 8.0) system. The TFA system: The presence of multiple basic amino acids in the target molecule contributes to its relatively large polarity. After the crude peptide product was completely dissolved in water by ultrasonication, the solution was filtered through a 0.22 µm membrane, and the filtrate was then separated using a WATERS Prep150 HPLC system with mobile phases A (0.1% trifluoroacetic acid and 10% acetonitrile in water, v/v) and B (0.1% trifluoroacetic acid and 90% acetonitrile in water, v/v). The chromatography column was an X-SELECT OBD C-18 (WATERS, 10 µm, 19 × 250 mm) reversed-phase chromatography column. During purification, the detection wavelength of the chromatograph was set to 220 nm, and the flow rate was 15 mL/min. The product-related fraction was collected, lyophilized, and then secondarily purified with a 25 mM ammonium bicarbonate (pH 8.0) system. The product obtained from the first-step purification was dissolved in a 25 mM ammonium bicarbonate buffer and then loaded onto a column; the ammonium bicarbonate system: the mobile phases were A (25 mM ammonium bicarbonate in water) and B (100% acetonitrile). The chromatography column was an X-SELECT OBD C-18 (WATERS, 5 µm, 19 × 250 mm) reversed-phase chromatography column. During purification, the detection wavelength of the chromatograph was set to 220 nm, and the flow rate was 10 mL/min. The product-related fraction was collected and lyophilized to give a purified product of polypeptide compound 14, with a yield of about 20%. The compound purity of the purified polypeptide product was determined through a WATERS H-CLASS analytical ultra-high performance liquid chromatography system (chromatography column: ReproSil-Pur Basic C-18 (Dr. Maisch, 5 µm, 4.6 × 150 mm), and its purity was 97.92%. The molecular weight of the compound was confirmed through an Agilent Q-TOF 6530 system (chromatography column: ReproSil-Pur Basic C-18 (Dr. Maisch, 5 µm, 4.6 × 150 mm), and the ion current shown in the mass spectrum was: 815.4899 [M+3H]³⁺/3.

### 3.2. Chemical synthesis of polypeptide compound 15

y-G-r-k-k-r-r-q-r-r-r-AEEA-Tle-Tle-T-D-V (SEQ ID NO. 15)

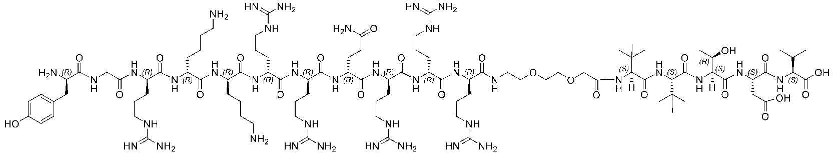

The synthesis of polypeptide compound 15 was carried out with reference to the synthesis of polypeptide compound 14 described above, with the difference being that the condensation sequence of amino acid derivatives was as follows: Fmoc-Asp(OtBu)-OH, Fmoc-Thr(tBu)-OH, Fmoc-Tle-OH, Fmoc-Tle-OH, Fmoc-AEEA-OH, Fmoc-D-Arg(Pbf)-OH, Fmoc-D-Arg(Pbf)-OH, Fmoc-D-Arg(Pbf)-OH, Fmoc-D-Gln(Trt)-OH, Fmoc-D-Arg(Pbf)-OH, Fmoc-D-Arg(Pbf)-OH, Fmoc-D-Lys(Boc)-OH, Fmoc-D-Lys(Boc)-OH, Fmoc-D-Arg(Pbf)-OH, Fmoc-Gly-OH, and Fmoc-D-Tyr(tBu)-OH. The compound purity of the purified polypeptide product was determined through a WATERS H-CLASS analytical ultra-high performance liquid chromatography system (chromatography column: ReproSil-Pur Basic C-18 (Dr. Maisch, 5 µm, 4.6 × 150 mm)), and its purity was 98.73%. The molecular weight of the compound was confirmed through an Agilent Q-TOF 6530 system (chromatography column: ReproSil-Pur Basic C-18 (Dr. Maisch, 5 µm, 4.6 × 150 mm), and the ion current shown in the mass spectrum was: 749.4533 [M+3H]³⁺/3.

### 3.3. Chemical synthesis of polypeptide compound 16

y-G-r-k-k-r-r-q-r-r-r-Nle-T-T-Tle-Tle-T-D-V (SEQ ID NO. 16)

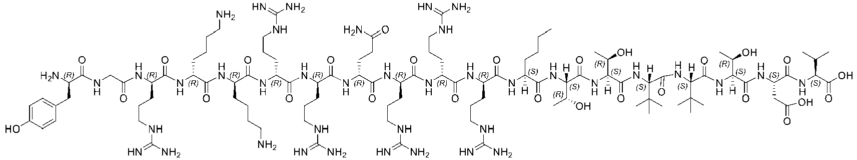

The synthesis of polypeptide compound 16 was carried out with reference to the synthesis of polypeptide compound 14 described above, with the difference being that the condensation sequence of amino acid derivatives was as follows: Fmoc-Asp(OtBu)-OH, Fmoc-Thr(tBu)-OH, Fmoc-Tle-OH, Fmoc-Tle-OH, Fmoc-Thr(tBu)-OH, Fmoc-Thr(tBu)-OH, Fmoc-Nle-OH, Fmoc-D-Arg(Pbf)-OH, Fmoc-D-Arg(Pbf)-OH, Fmoc-D-Arg(Pbf)-OH, Fmoc-D-Gln(Trt)-OH, Fmoc-D-Arg(Pbf)-OH, Fmoc-D-Arg(Pbf)-OH, Fmoc-D-Lys(Boc)-OH, Fmoc-D-Lys(Boc)-OH, Fmoc-D-Arg(Pbf)-OH, Fmoc-Gly-OH, and Fmoc-D-Tyr(tBu)-OH. The compound purity of the purified polypeptide product was determined through a WATERS H-CLASS analytical ultra-high performance liquid chromatography system (chromatography column: ReproSil-Pur Basic C-18 (Dr. Maisch, 5 µm, 4.6 × 150 mm), and its purity was 97.93%. The molecular weight of the compound was confirmed through an Agilent Q-TOF 6530 system (chromatography column: ReproSil-Pur Basic C-18 (Dr. Maisch, 5 µm, 4.6 × 150 mm), and the ion current shown in the mass spectrum was: 806.1549 [M+3H]³⁺/3.

### 3.4. Chemical synthesis of polypeptide compound 17

y-G-r-k-k-r-r-q-r-r-r-T-homoArg-homoArg-I-T-D-V (SEQ ID NO. 17)

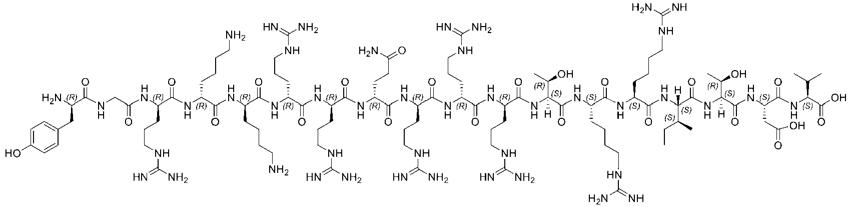

The synthesis of polypeptide compound 17 was carried out with reference to the synthesis of polypeptide compound 14 described above, with the difference being that the condensation sequence of amino acid derivatives was as follows: Fmoc-Asp(OtBu)-OH, Fmoc-Thr(tBu)-OH, Fmoc-Ile-OH, Fmoc-homoArg(Pbf)-OH, Fmoc-homoArg(Pbf)-OH, Fmoc-Thr(tBu)-OH, Fmoc-D-Arg(Pbf)-OH, Fmoc-D-Arg(Pbf)-OH, Fmoc-D-Arg(Pbf)-OH, Fmoc-D-Gln(Trt)-OH, Fmoc-D-Arg(Pbf)-OH, Fmoc-D-Arg(Pbf)-OH, Fmoc-D-Lys(Boc)-OH, Fmoc-D-Lys(Boc)-OH, Fmoc-D-Arg(Pbf)-OH, Fmoc-Gly-OH, and Fmoc-D-Tyr(tBu)-OH. The compound purity of the purified polypeptide product was determined through a WATERS H-CLASS analytical ultra-high performance liquid chromatography system (chromatography column: ReproSil-Pur Basic C-18 (Dr. Maisch, 5 µm, 4.6 × 150 mm), and its purity was 98.77%. The molecular weight of the compound was confirmed through an Agilent Q-TOF 6530 system (chromatography column: ReproSil-Pur Basic C-18 (Dr. Maisch, 5 µm, 4.6 × 150 mm), and the ion current shown in the mass spectrum was: 810.4980 [M+3H]³⁺/3.

### 3.5. Chemical synthesis of polypeptide compound 18

y-G-r-k-k-r-r-q-r-r-r-G-Aib-Dab-homoArg-Tle-Tle-T-D-V (SEQ ID NO. 18)

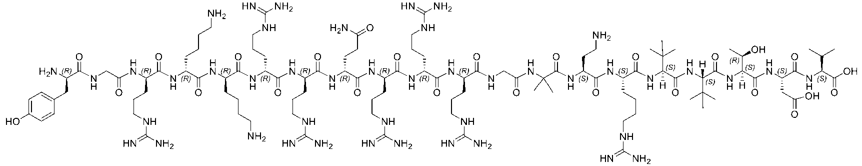

The synthesis of polypeptide compound 18 was carried out with reference to the synthesis of polypeptide compound 14 described above, with the difference being that the condensation sequence of amino acid derivatives was as follows: Fmoc-Asp(OtBu)-OH, Fmoc-Thr(tBu)-OH, Fmoc-Tle-OH, Fmoc-Tle-OH, Fmoc-homoArg(Pbf)-OH, Fmoc-Dab(Boc)-OH, Fmoc-Aib-OH, Fmoc-Gly-OH, Fmoc-D-Arg(Pbf)-OH, Fmoc-D-Arg(Pbf)-OH, Fmoc-D-Arg(Pbf)-OH, Fmoc-D-Gln(Trt)-OH, Fmoc-D-Arg(Pbf)-OH, Fmoc-D-Arg(Pbf)-OH, Fmoc-D-Lys(Boc)-OH, Fmoc-D-Lys(Boc)-OH, Fmoc-D-Arg(Pbf)-OH, Fmoc-Gly-OH, and Fmoc-D-Tyr(tBu)-OH. The compound purity of the purified polypeptide product was determined through a WATERS H-CLASS analytical ultra-high performance liquid chromatography system (chromatography column: ReproSil-Pur Basic C-18 (Dr. Maisch, 5 µm, 4.6 × 150 mm), and its purity was 98.68%. The molecular weight of the compound was confirmed through an Agilent Q-TOF 6530 system (chromatography column: ReproSil-Pur Basic C-18 (Dr. Maisch, 5 µm, 4.6 × 150 mm), and the ion current shown in the mass spectrum was: 838.5120 [M+3H]³⁺/3.

### 3.6. Chemical synthesis of polypeptide compound 19

y-G-r-k-k-r-r-q-r-r-r-G-Aib-Dab-T-Chg-Tle-S-D-V (SEQ ID NO. 19)

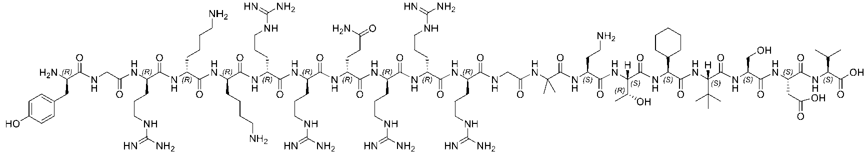

The synthesis of polypeptide compound 19 was carried out with reference to the synthesis of polypeptide compound 14 described above, with the difference being that the condensation sequence of amino acid derivatives was as follows: Fmoc-Asp(OtBu)-OH, Fmoc-Ser(tBu)-OH, Fmoc-Tle-OH, Fmoc-Chg-OH, Fmoc-Thr(tBu)-OH, Fmoc-Dab(Boc)-OH, Fmoc-Aib-OH, Fmoc-Gly-OH, Fmoc-D-Arg(Pbf)-OH, Fmoc-D-Arg(Pbf)-OH, Fmoc-D-Arg(Pbf)-OH, Fmoc-D-Gln(Trt)-OH, Fmoc-D-Arg(Pbf)-OH, Fmoc-D-Arg(Pbf)-OH, Fmoc-D-Lys(Boc)-OH, Fmoc-D-Lys(Boc)-OH, Fmoc-D-Arg(Pbf)-OH, Fmoc-Gly-OH, and Fmoc-D-Tyr(tBu)-OH. The compound purity of the purified polypeptide product was determined through a WATERS H-CLASS analytical ultra-high performance liquid chromatography system (chromatography column: ReproSil-Pur Basic C-18 (Dr. Maisch, 5 µm, 4.6 × 150 mm), and its purity was 98.95%. The molecular weight of the compound was confirmed through an Agilent Q-TOF 6530 system (chromatography column: ReproSil-Pur Basic C-18 (Dr. Maisch, 5 µm, 4.6 × 150 mm), and the ion current shown in the mass spectrum was: 819.4897 [M+3H]³⁺/3.

### 3.7. Chemical synthesis of polypeptide compound 20

y-G-r-k-k-r-r-q-r-r-r-G-T-Tle-Tle-T-D-V (SEQ ID NO. 20)

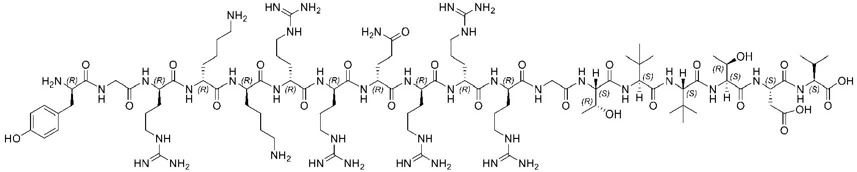

The synthesis of polypeptide compound 20 was carried out with reference to the synthesis of polypeptide compound 14 described above, with the difference being that the condensation sequence of amino acid derivatives was as follows: Fmoc-Asp(OtBu)-OH, Fmoc-Thr(tBu)-OH, Fmoc-Tle-OH, Fmoc-Tle-OH, Fmoc-Thr(tBu)-OH, Fmoc-Gly-OH, Fmoc-D-Arg(Pbf)-OH, Fmoc-D-Arg(Pbf)-OH, Fmoc-D-Arg(Pbf)-OH, Fmoc-D-Gln(Trt)-OH, Fmoc-D-Arg(Pbf)-OH, Fmoc-D-Arg(Pbf)-OH, Fmoc-D-Lys(Boc)-OH, Fmoc-D-Lys(Boc)-OH, Fmoc-D-Arg(Pbf)-OH, Fmoc-Gly-OH, and Fmoc-D-Tyr(tBu)-OH. The compound purity of the purified polypeptide product was determined through a WATERS H-CLASS analytical ultra-high performance liquid chromatography system (chromatography column: ReproSil-Pur Basic C-18 (Dr. Maisch, 5 µm, 4.6 × 150 mm), and its purity was 97.06%. The molecular weight of the compound was confirmed through an Agilent Q-TOF 6530 system (chromatography column: ReproSil-Pur Basic C-18 (Dr. Maisch, 5 µm, 4.6 × 150 mm), and the ion current shown in the mass spectrum was: 753.7843 [M+3H]³⁺/3.

### 3.8. Chemical synthesis of polypeptide compound 21

y-G-r-k-k-r-r-q-r-r-r-G-T-Chg-Tle-S-D-V (SEQ ID NO. 21)

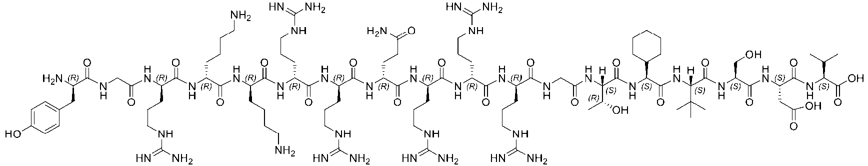

The synthesis of polypeptide compound 21 was carried out with reference to the synthesis of polypeptide compound 14 described above, with the difference being that the condensation sequence of amino acid derivatives was as follows: Fmoc-Asp(OtBu)-OH, Fmoc-Ser(tBu)-OH, Fmoc-Tle-OH, Fmoc-Chg-OH, Fmoc-Thr(tBu)-OH, Fmoc-Gly-OH, Fmoc-D-Arg(Pbf)-OH, Fmoc-D-Arg(Pbf)-OH, Fmoc-D-Arg(Pbf)-OH, Fmoc-D-Gln(Trt)-OH, Fmoc-D-Arg(Pbf)-OH, Fmoc-D-Arg(Pbf)-OH, Fmoc-D-Lys(Boc)-OH, Fmoc-D-Lys(Boc)-OH, Fmoc-D-Arg(Pbf)-OH, Fmoc-Gly-OH, and Fmoc-D-Tyr(tBu)-OH. The compound purity of the purified polypeptide product was determined through a WATERS H-CLASS analytical ultra-high performance liquid chromatography system (chromatography column: ReproSil-Pur Basic C-18 (Dr. Maisch, 5 µm, 4.6 × 150 mm), and its purity was 98.54%. The molecular weight of the compound was confirmed through an Agilent Q-TOF 6530 system (chromatography column: ReproSil-Pur Basic C-18 (Dr. Maisch, 5 µm, 4.6 × 150 mm), and the ion current shown in the mass spectrum was: 757.7843 [M+3H]³⁺/3.

### 3.9. Chemical synthesis of polypeptide compound 22

r-K-K-R-r-Q-R-R-r-G-Aib-Dab-T-Tle-Tle-T-D-V (SEQ ID NO. 22)

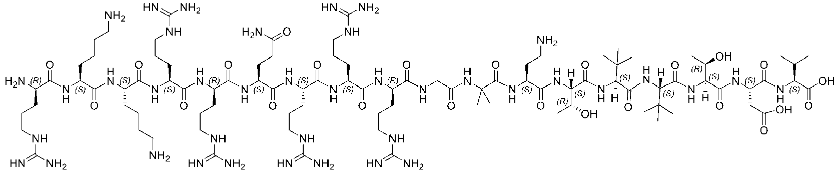

The synthesis of polypeptide compound 22 was carried out with reference to the synthesis of polypeptide compound 14 described above, with the difference being that the condensation sequence of amino acid derivatives was as follows: Fmoc-Asp(OtBu)-OH, Fmoc-Thr(tBu)-OH, Fmoc-Tle-OH, Fmoc-Tle-OH, Fmoc-Thr(tBu)-OH, Fmoc-Dab(Boc)-OH, Fmoc-Aib-OH, Fmoc-Gly-OH, Fmoc-D-Arg(Pbf)-OH, Fmoc-Arg(Pbf)-OH, Fmoc-Arg(Pbf)-OH, Fmoc-Gln(Trt)-OH, Fmoc-D-Arg(Pbf)-OH, Fmoc-Arg(Pbf)-OH, Fmoc-Lys(Boc)-OH, Fmoc-Lys(Boc)-OH, and Fmoc-D-Arg(Pbf)-OH. The compound purity of the purified polypeptide product was determined through a WATERS H-CLASS analytical ultra-high performance liquid chromatography system (chromatography column: ReproSil-Pur Basic C-18 (Dr. Maisch, 5 µm, 4.6 × 150 mm), and its purity was 97.82%. The molecular weight of the compound was confirmed through an Agilent Q-TOF 6530 system (chromatography column: ReproSil-Pur Basic C-18 (Dr. Maisch, 5 µm, 4.6 × 150 mm), and the ion current shown in the mass spectrum was: 742.1292 [M+3H]³⁺/3.

### Biological Tests and Evaluations

The present disclosure is further described and explained below with reference to test examples. However, these examples are not intended to limit the scope of the present disclosure.

### 1. Reagents

**Table 1-3**

| No. | Reagent | Source |
|---|---|---|
| 1 | Phosphate-buffered saline (PBS) | Gibco 10010023 |
| 2 | Tetrazolium chloride | Phygene |
| 3 | Isopropyl thiogalactopyranoside (IPTG) | Sigma-Aldrich |
| 4 | Bovine serum albumin (BSA) | Sigma-Aldrich |
| 5 | Imidazole | Sigma-Aldrich |
| 6 | Polysorbate-20 (Tween 20) | Sigma-Aldrich |
| 7 | Tetramethylbenzidine (TMB chromogenic solution) | Sigma-Aldrich |
| 8 | TEV protease | New England Biolabs |
| 9 | Biotin ligase | Genecopoeia |
| 10 | Methanol | Fisher |
| 11 | Formic acid | Sigma-Aldrich |
| 12 | Alteplase | Boehringer Ingelheim |
| 13 | Acetonitrile | Fisher |
| 14 | Ammonium acetate | Sigma-Aldrich |
| 15 | Urea assay kit | Guilin URIT Medical Electronic Co., Ltd. |
| 16 | Creatinine assay kit | Guilin URIT Medical Electronic Co., Ltd. |

### 2. Instruments

**Table 1-4**

| No. | Instrument | Source |
|---|---|---|
| 1 | SpectraMax M5 microplate reader | Molecular Devices |
| 2 | Dual-channel injection pump | Jiaxing Biotaor Instrument Co., Ltd. |
| 3 | BioPac physiological signal recorder | BIOPAC Systems, Inc. |
| 4 | 405 LS plate washer | Porton, USA |
| 5 | AKTA pure 150 purification system | General Electric Company |
| 6 | LYNX 4000 high-speed centrifuge | Thermo Fisher |
| 7 | Electrophoresis apparatus | Thermo Fisher |
| 8 | Protein staining and destaining system | Genscript |
| 9 | IS-RDS6 stackable shaker | Crystal Technology & Industries |
| 10 | AH-1500 high-pressure homogenizer | ATS |
| 11 | Triple Quad 4500 tandem quadrupole liquid chromatography-mass spectrometry system | SCIEX |
| 12 | Triple Quad 6500 tandem quadrupole liquid chromatography-mass spectrometry system | SCIEX |
| 13 | URIT-8400 fully automatic biochemical analyzer | Guilin URIT Medical Electronic Co., Ltd. |

### 3. Test examples

### 3.1. Assessment of affinities of polypeptide compounds for human PSD-95

### 3.1.1. Objective

The objective of this test example was to determine the affinities of polypeptide compounds for human PSD95-PDZ2 using competitive ELISAs.

### 3.1.2. Expression and purification of human PSD95-PDZ2 protein

The amino acid sequence of PDZ2 protein was designed using human PSD95 (Uniprot Entry: P78352) as a template for PDZ: PDZ2 (155-249, the Flag-His-Avitag-TEVsite tag is italicized and was subsequently used for purification and biotinylation): *MDYKDDDDKGSHHHHHHHHGGGGSGGGGSGL NDIFEAQKIEWHEGGGGSENLYFQGGGGGS*AEKVMEIKLIKGPKGLGFSIAGG VGNQHIPGDNSIYVTKIIEGGAAHKDGRLQIGDKILAVNSVGLEDVMHEDAVA

ALKNTYDVVYLKVAKPSNA (SEQ ID NO. 62). The PDZ2 gene was constructed onto a PET expression vector, and induced expression was performed using BL21-DE3 *E. coli* cells under conditions of 37 °C and 1 mM IPTG 4 h. Then centrifugation was performed at 10,000 g at 4 °C for 10 min, and the cell pellets were collected, resuspended in 1× PBS solution, and then lysed using a homogenizer. The lysate was centrifuged at high speed, and the supernatant was collected and filtered through a 0.45 µm membrane. A Ni-Sepharose affinity column was equilibrated with 5 column volumes of a 20 mM phosphate buffer (pH 8.0). The sample was centrifuged at high speed to remove impurities and loaded onto the column for binding. The column was rinsed with the 20 mM phosphate buffer until the A280 reading dropped to the baseline. Gradient elution was then performed with the 20 mM phosphate buffer and 0-500 mM imidazole. A protein was collected, and whether it was the target protein was determined. The purified sample described above was buffer-exchanged into 1× PBS solution, and the resulting solution was concentrated to a volume of 2 mL and further purified using a Superdex200 gel chromatography column (GE) equilibrated with 1× PBS. The target peak was collected and aliquoted for later use.

### 3.1.3. Method

Site-specifically biotinylated Biotin-NA-1 (sequence: Biotin-YGRKKRRQRRRKLSSIESDV; SEQ ID NO. 63) was used for competitive ELISA binding assays. In this test example, the positive controls were NA-1 (sequence: YGRKKRRQR RRKLSSIESDV; SEQ ID NO. 64) and NoNO42 (sequence: YGrKKRrQrRRkLSSIESDV; SEQ ID NO. 65), and the negative control was NA-1_{(ADA)} (sequence: YGRKKRRQRRRKLSSIEADA; SEQ ID NO. 66), which is a mutant of NA-1 obtained by mutating the amino acids at positions 0 and -2 into Ala and has been shown to have no ability to bind to PDZ2 (Science, 2002, 298:846-50). PSD95-PDZ2 (the tag was removed by TEV enzyme) was diluted with 1× PBS buffer to 1 µg/mL, added to a 96-well microplate (Corning, 9018, 25/box 96-well clear flat bottom plate) at 100 µL/well, and left to stand overnight at 4 °C for 16-20 h. After the liquid was discarded, the plate was washed three times with PBST (pH 7.4, 0.05% Tween-20) buffer, and a 4% BSA blocking solution diluted with PBST buffer was then added (300 µL/well). The plate was blocked by incubation in an incubator at 37 °C for 1 h. After the blocking was complete, the blocking solution was discarded, and the plate was washed three times with PBST buffer. Biotin-NA-1, at a constant concentration of 0.3 µM, and the test compounds, having a starting concentration of 100 µM and serially diluted ten-fold with PBS buffer to seven concentrations (100, 10, 1, 0.1, 0.01, 0.001, 0.0001, and 0 µM), were then added, and the plate was incubated in an incubator at 37 °C for 1 h. After the incubation was complete, the reaction solutions in the microplate were discarded, and the plate was washed three times with PBST. 100 µL of HRP-SA secondary antibody (diluted at 1:2000) was added to each well, and the plate was incubated at 37 °C for 1 h. After the plate was washed three times with PBST, 100 µL of TMB chromogenic substrate was added. The plate was incubated at room temperature for 1-3 min, and 100 µL of 1 M sulfuric acid was added to stop the reaction.

### 3.1.4. Sample analysis and data processing

The absorbance at 450 nm was measured using a SpectraMax M5 microplate reader, and non-linear fitting was performed using GraphPad Prism 9 to calculate the binding IC₅₀ values of the test compounds to PSD95-PDZ2 protein. Specific data are shown in Table 1.

**Table 1-5. The abilities of polypeptide compounds to bind to human PSD95-PDZ2**

| Polypeptide compound | IC₅₀ (µM)* | rIC₅₀** |
|---|---|---|
| NA-1 | 0.909 | 1 |
| NoNO42 | 0.917 | 1.01 |
| 14 | 0.713 | 0.78 |
| 15 | 2.619(NA-1:0.872) | 3.00 |
| 16 | 1.517(NA-1:0.872) | 1.74 |
| 17 | 0.605(NA-1:0.872) | 0.69 |
| 18 | 0.152 | 0.17 |
| 19 | 0.612 | 0.67 |
| 20 | 0.763 | 0.84 |
| 21 | 0.800 | 0.88 |
| 22 | 0.881(NA-1:0.517) | 1.70 |

| | | |
|---|---|---|
| * The error of this test was within three times. ** rIC₅₀ represents the ratio of the affinity of the polypeptide compound to the affinity of NA-1. | | |

### 3.1.5. Conclusion:

The results show that the compounds of the present disclosure all effectively bound to the target protein human PSD95-PDZ2. The fact that the PSD95-PDZ domains are highly conserved across different species facilitates subsequent animal evaluations.

### 3.2. Assessment of binding specificity of polypeptide compounds for human PSD95-PDZ2

### 3.2.1. Objective

The three PDZ domains in the PSD95 protein are structurally similar. Binding to the PDZ1 and PDZ2 domains can effectively block PSD95-mediated neuroexcitotoxicity. However, the biological function of binding to the PDZ3 domain is unclear. Therefore, to avoid potential safety concerns, this test example assessed the selectivity of polypeptide compounds for the PDZ2 and PDZ3 domains, aiming to identify polypeptide compounds that specifically bind to the PDZ2 domain.

### 3.2.2. Expression and purification of human PSD95-PDZ3 protein

The expression and purification of PSD95-PDZ3 protein were carried out with reference to the steps in 3.1.2. The amino acid sequence of PDZ3 protein: PDZ3 (309-401, the Flag-His-Avitag-TEVsite tag is italicized and was subsequently used for purification and biotinylation):

MDYKDDDDKGSHHHHHHHHGGGGSGGGGSGLNDIF
EAQKIEWHEGGGGSENLYFQGGGGGSREPRRIVIHRGSTGLGFNIVGGEDGEGI
FISFILAGGPADLSGELRKGDQILSVNGVDLRNASHEQAAIALKNAGQTVTIIA
QYKPEEYSRFE; SEQ ID NO. 67.

### 3.2.3. Method

Site-specifically biotinylated Biotin-PDZ2 and Biotin-PDZ3 proteins were used for binding ELISA assays. In this test example, the positive controls were NA-1 and NoNO42 (WO2022150655), and the negative control was NA-1_{(ADA)}. The test compounds were diluted with 1× PBS buffer to 2 µM, added to a 96-well microplate (Corning, 9018 25/box 96-well clear flat bottom plate) at 100 µL/well, and left to stand overnight at 4 °C for 16-20 h. After the liquid was discarded, the plate was washed three times with PBST (pH 7.4, 0.05% Tween-20) buffer, and a 4% BSA blocking solution diluted with PBST buffer was then added (300 µL/well). The plate was blocked by incubation in an incubator at 37 °C for 1 h. After the blocking was complete, the blocking solution was discarded, and the plate was washed three times with PBST buffer. Biotin-PDZ2 or Biotin-PDZ3, having a starting concentration of 10 µM and serially diluted ten-fold with 1× PBS buffer to seven concentrations (10, 1, 0.1, 0.01, 0.001, 0.0001, 0.00001, and 0 µM), was then added, and the plate was incubated in an incubator at 37 °C for 1 h. After the incubation was complete, the reaction solutions in the microplate were discarded, and the plate was washed three times with PBST. 100 µL of HRP-SA secondary antibody (diluted at 1:2000) was added to each well, and the plate was incubated at 37 °C for 1 h. After the plate was washed three times with PBST, 100 µL of TMB chromogenic substrate was added. The plate was incubated at room temperature for 1-3 min, and 100 µL of 1 M sulfuric acid was added to stop the reaction.

### 3.2.4. Sample analysis and data processing

The absorbance at 450 nm was measured using a SpectraMax M5 microplate reader, and the binding EC50 values of the test compounds to Biotin-PDZ2 or Biotin-PDZ3 were calculated using GraphPad Prism 9. Specific data are shown in Tables 2-1 and 2-2.

**Table 2-1. The abilities of polypeptide compounds to bind to human PSD95-PDZ2 and human PSD95-PDZ3**

| Polypeptide compound | EC₅₀ (PDZ2, µM) | EC₅₀ (PDZ3, µM) | Specificity for PDZ2 (PDZ3/PDZ2) |
|---|---|---|---|
| NA-1 | 0.083 | >1000 | >10000 |
| NoNO42 | 0.098 | >1000 | >10000 |
| 14 | 0.076 | >1000 | **>10000** |
| 15 | 0.088 | >1000 | **>10000** |
| 16 | 0.081 | >1000 | **>10000** |
| 17 | 0.269 | 38.26 | 142 |
| 18 | 0.015 | 1.19 | 79 |
| 19 | 0.029 | >1000 | **>10000** |
| 20 | 0.015 | 3.23 | 215 |
| 21 | 0.018 | >1000 | **>10000** |

**Table 2-2. The abilities of polypeptide compounds to bind to human PSD95-PDZ2 and human PSD95-PDZ3**

| Polypeptide compound | Specificity for PDZ2 (PDZ3/PDZ2) |
|---|---|
| NA-1 | >10000 |
| 22 | >10000 |

### 3.2.5. Conclusion

The results show that the compounds of the present disclosure all selectively bound to the PSD95-PDZ2 domain, with the selectivity of polypeptide compounds 14, 15, 16, 19, 21, and 22 being comparable to that of the positive drugs NA-1 and NoNO42.

### 3.3. Assessment of hemolysis risk of polypeptide compound in rat, rabbit, and human whole blood

Hemolysis refers to the phenomenon in which the membrane of erythrocytes is destroyed, along with increased transparency and a dark red appearance. Certain pharmaceutical ingredients, excipients, etc. may contain hemolytic components, which may cause hemolysis in the human body and cause adverse reactions such as local swelling and blood circulation dysfunction. On the basis of the principle that the hemoglobin released from lysed red blood cells has absorption in the visible light wave band, test compound solutions were added to rat red blood cell suspensions, and after incubation, the degrees of hemolysis were determined using a microplate reader.

### 3.3.1. Objective

This test example assessed whether a polypeptide compound causes hemolytic reactions in rat, rabbit, and human whole blood.

### 3.3.2. Method

Preparation of red blood cell suspension: 100 µL of fresh whole blood was collected, and 900 µL of 1× PBS solution was then added. The mixture was shaken on a plate shaker at 30 rpm for 5 min and then centrifuged at 1000 g for 5 min, and the supernatant was discarded. The above washing step was repeated until the supernatant no longer appeared red. The suspension was set aside for later use.

Preparation of test polypeptide solutions: An appropriate amount of 1× PBS solution was added to a powdered polypeptide, and the polypeptide was dissolved to form a standard stock solution. The stock solution was then diluted with 1× PBS to form test solutions with concentrations of 1, 3, 10, 30, 100, and 300 µg/mL, and two replicate wells were set for each concentration. Meanwhile, blank 1× PBS was used as a negative control, and a 1× PBS solution containing 0.1% Triton X-100 was used as a positive control.

Incubation: A test solution (500 µL) was added to the red blood cell suspension, and then the mixture was well shaken on a plate shaker at 30 rpm for 5 min, incubated at 37 °C for 1 h, and centrifuged at 1000 g for 5 min.

### 3.3.3. Sample analysis and data processing

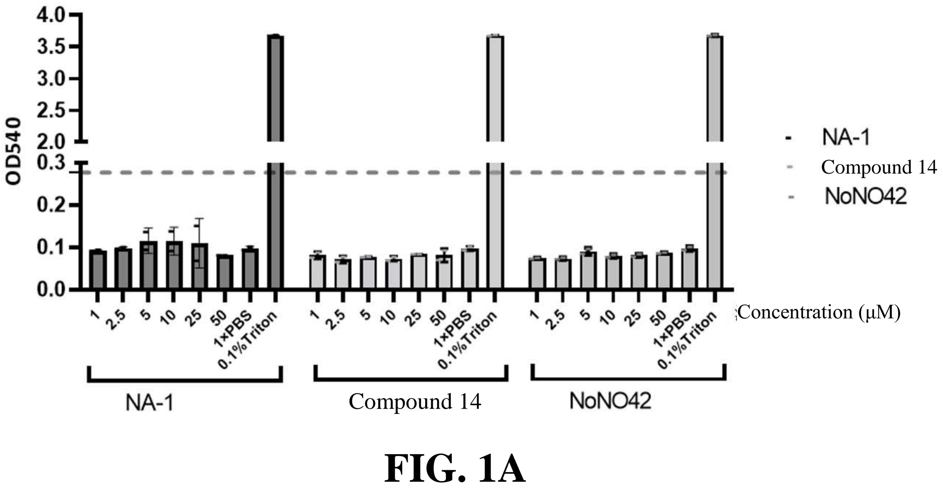

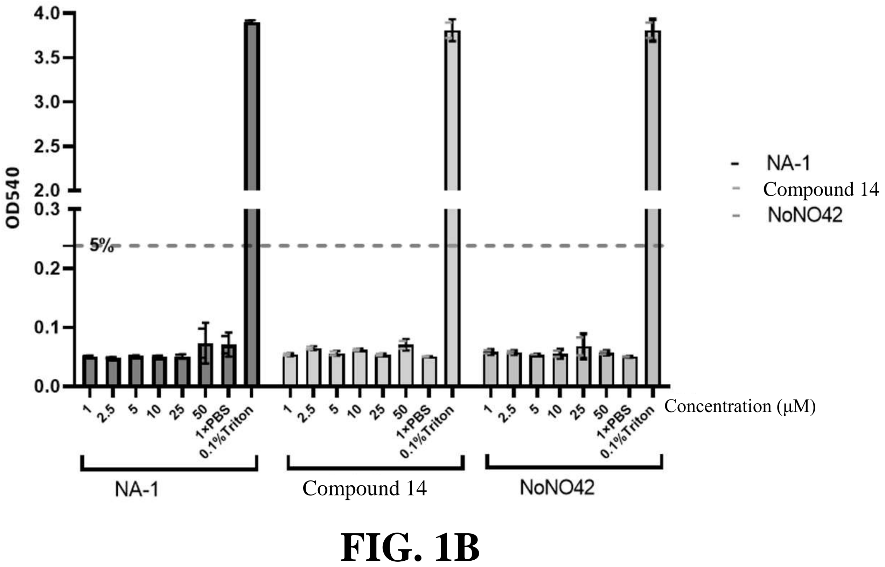

100 µL of the supernatant was transferred to a single well of a microplate. The absorbance at 540 nm was measured using a SpectraMax M5 microplate reader, and data analysis was performed using GraphPad Prism 9. The hemolysis rate (%) was calculated using the formula: (test sample absorbance - negative control absorbance) / (positive control absorbance - negative control absorbance) × 100%. A rate of less than 5% indicates the absence of hemolysis, while a rate of greater than 5% indicates the presence of hemolysis. The experimental results are shown in Table 3, FIG. 1A, and FIG. 1B.

**Table 3. Testing of the hemolysis risk of the polypeptide compound**

| Species | Polypeptide compound | Hemolysis rate (%) | | | | | |
|---|---|---|---|---|---|---|---|
| | | 1.0 µM | 2.5 µM | 5.0 µM | 10 µM | 25 µM | 50 µM |
| Rat | NA-1 | -0.12 | 0.03 | 0.50 | 0.48 | 0.34 | -0.46 |
| | NoNO42 | 0.25 | 0.10 | 0.06 | 0.36 | -0.10 | -0.09 |
| | 14 | 0.11 | 0.17 | -0.11 | 0.00 | -0.16 | 0.12 |
| Human | NA-1 | -0.52 | -0.59 | -0.49 | -0.54 | -0.53 | 0.06 |
| | NoNO42 | 0.21 | 0.19 | 0.08 | 0.13 | 0.46 | 0.17 |
| | 14 | 0.09 | 0.38 | 0.15 | 0.31 | 0.08 | 0.53 |

### 3.3.4. Conclusion:

The results show that in rat and human whole blood, polypeptide compound 14 and the positive drugs NA-1 and NoNO42 all exhibited hemolysis rates of less than 5% across the concentration range of 1 to 50 µM, indicating no hemolysis risk.

### 3.3.5. Assessment of hemolysis risk of polypeptide compounds in rabbit whole blood

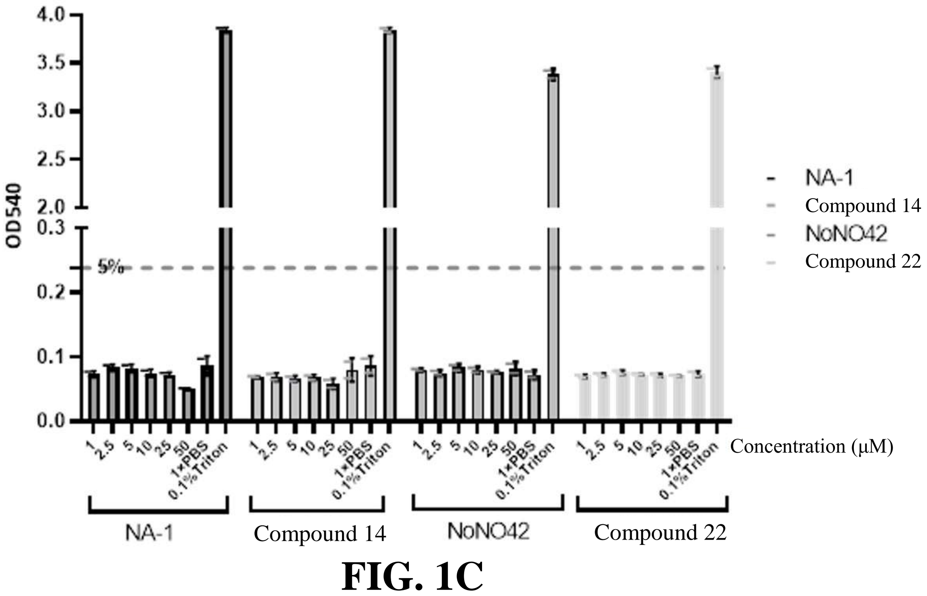

A method similar to that in section 3.3.2 was adopted, with the difference being that this test example used fresh rabbit whole blood instead. The experimental results are shown in FIG. 1C. The results show that in rabbit whole blood, polypeptide compounds 14 and 22 and the positive drugs NA-1 and NoNO42 all exhibited hemolysis rates of less than 5% across the concentration range of 1 to 50 µM, indicating no hemolysis risk.

### 3.4. Assessment of in vitro stability of polypeptide compounds in rat and human plasma

Plasma contains various hydrolases. These hydrolases can decompose and metabolize drug molecules, leading to a rapid decline in the concentration of drug molecules in plasma, failure to reach effective concentrations, relatively high clearance rates, relatively short half-lives, and poor pharmacokinetic and pharmacodynamic profiles. Therefore, plasma stability serves as an important indicator of druggability.

### 3.4.1. Objective

This test example assessed the *in vitro* stability of polypeptide compounds in rat and human plasma, and is divided into two parts: 1) the stability of the individual polypeptide compounds in rat and human plasma; and 2) the stability of the polypeptide compounds in rat and human plasma when the polypeptide compounds are administered simultaneously with alteplase (rt-PA).

### 3.4.2. Method

Solution preparation: An appropriate amount of a test polypeptide compound was weighed out and dissolved in an appropriate amount of 1× PBS to form a 1 mM test solution. NA-1 and NoNO42 were used as positive drugs, propantheline bromide was used as a human plasma test control, and lovastatin was used as a rat plasma test control.

Plasma stability test: 12.5 µL of the 1 mM test solution was added to a 495 µL pre-incubated plasma sample. The prepared plasma sample was added to centrifuge tubes of different incubation times (0 min, 10 min, 20 min, 30 min, 60 min, and 120 min) (50 µL/tube), and the tubes were then incubated in a water bath at 37 °C with shaking at 60 rpm. For each incubation time, two parallel samples were set. Subsequently, 200 µL of stop solution was added to stop the incubation, and the mixtures were vortexed for 5 min. Then the mixtures were centrifuged at 10,000 rpm at 4 °C for 10 min to remove the protein in the sample, and 70 µL of the supernatant was transferred to a new 96-well plate (70 µL of water was added to each well beforehand) and well mixed.

Plasma stability test with alteplase added: An appropriate amount of solid alteplase was weighed out, and an appropriate volume of water was added. The mixture was vortexed to dissolve alteplase, forming a 1 mg/mL solution of alteplase. 250 µL of the 1 mg/mL solution of alteplase was added to 4750 µL of plasma to form a plasma sample containing 50 µg/mL alteplase. The other procedures were the same as in the aforementioned "plasma stability test", with the difference being that the plasma sample containing 50 µg/mL alteplase was used instead of plasma.

### 3.4.3. Sample analysis and data processing:

The remaining compound content at each time point was determined by LC-MS/MS, and the relative remaining compound contents at the other time points were calculated with the content at 0 min as a standard (100%).

Sample pretreatment: A 30 µL plasma sample was taken and added to 120 µL of a 5% solution of formic acid in methanol containing 1 ng/mL internal standard (verapamil). The mixture was vortexed for 5 min and centrifuged at low temperature at 10,000 rpm for 10 min. 70 µL of the supernatant was taken, and 70 µL of a 0.1% aqueous solution of formic acid was added and well mixed, and the mixture was injected into an LC-MS system for analysis.

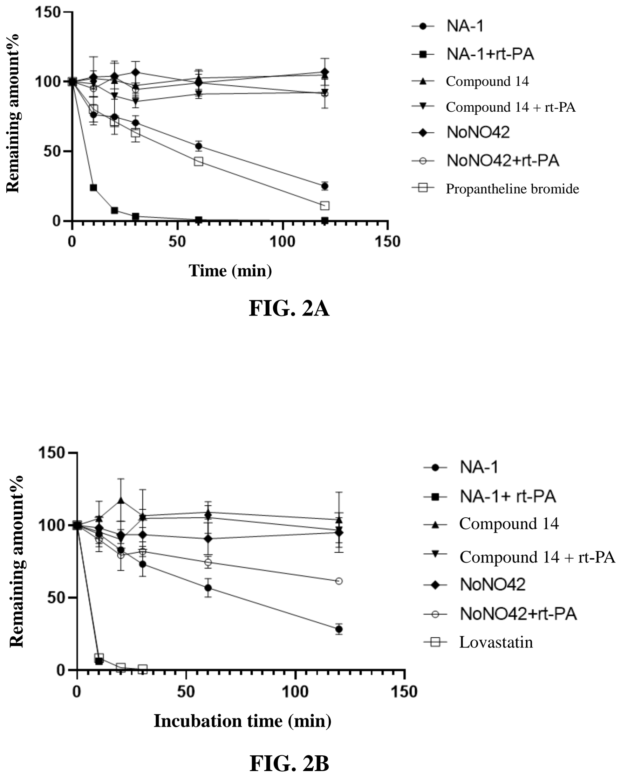

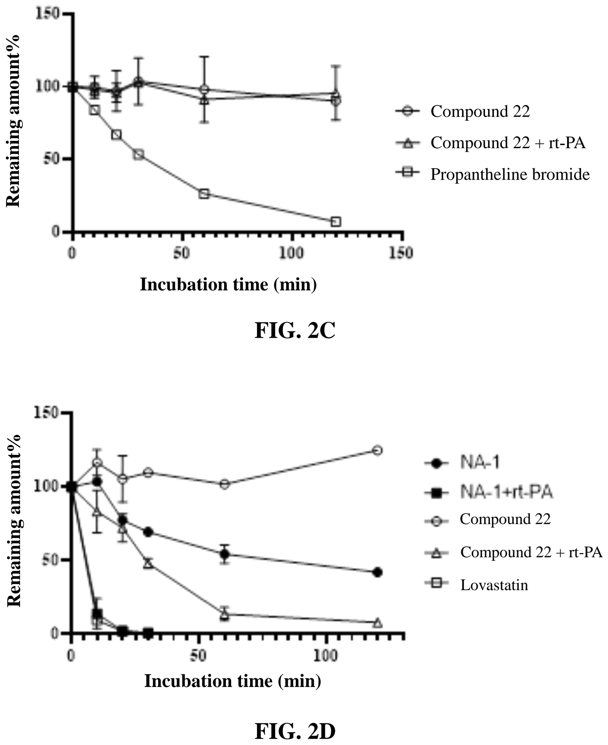

LC-MS analysis: (1) Chromatographic conditions: mobile phase A: 0.1% formic acid in water; mobile phase B: 0.1% formic acid in acetonitrile; flow rate: 0.5 mL/min; injection volume: 10 µL; chromatography column: nanomicro Unisil C18aq (4.6 mm × 150 mm, 5 µm); column temperature: 40 °C. (2) Mass-spectrometric conditions: The mass spectrometry analysis used an electrospray ionization (ESI) source, a positive ion analysis mode, and a multi-reaction monitoring (MRM) scan mode. The experimental results are shown in Table 4-1, Table 4-2, FIG. 2A, FIG. 2B, FIG. 2C, and FIG. 2D.

**Table 4-1. The plasma stability of the polypeptide compound**

| Species | Compound | Test concentration (µM) | Relative remaining compound content (%) | | | | | |
|---|---|---|---|---|---|---|---|---|
| | | | 0min | 10min | 20min | 30min | 60min | 120min |
| Human | Propantheline bromide | 2 | 100.0 | 80.2 | 71.5 | 63.4 | 42.7 | 10.9 |
| | NA-1 | 25 | 100.0 | 76.4 | 74.9 | 70.6 | 53.9 | 25.1 |
| | NA-1 +rt-PA | 25 | 100.0 | 23.9 | 7.6 | 3.4 | 0.9 | 0.3 |
| | NoNO42 | 25 | 100.0 | 103.5 | 104.1 | 106.8 | 99.3 | 107.1 |
| | NoNO42 +rt-PA | 25 | 100.0 | 95.0 | 103.4 | 94.5 | 99.2 | 91.4 |
| | Polypeptide compound 14 | 25 | 100.0 | 102.3 | 101.0 | 97.3 | 102.7 | 104.9 |
| | Polypeptide compound 14 + rt-PA | 25 | 100.0 | 98.6 | 89.8 | 85.8 | 91.2 | 92.3 |
| Rat | Lovastatin | 2 | 100.0 | 8.5 | 1.8 | 0.7 | NA | NA |
| | NA-1 | 25 | 100.0 | 94.1 | 83.0 | 73.3 | 57.0 | 28.4 |
| | NA-1 +rt-PA | 25 | 100.0 | 6.3 | NA | NA | NA | NA |
| | NoNO42 | 25 | 100.0 | 98.4 | 93.6 | 93.6 | 90.8 | 95.0 |
| | NoNO42 +rt-PA | 25 | 100.0 | 90.2 | 79.5 | 82.0 | 74.6 | 61.6 |
| | Polypeptide compound 14 | 25 | 100.0 | 104.8 | 117.4 | 106.7 | 109.1 | 103.9 |
| | Polypeptide compound 14 + rt-PA | 25 | 100.0 | 95.6 | 90.4 | 104.9 | 105.4 | 96.7 |

**Table 4-2. The plasma stability of the polypeptide compound**

| Species | Compound | Test concentration (µM) | Relative remaining compound content (%) | | | | | |
|---|---|---|---|---|---|---|---|---|
| | | | 0min | 10min | 20min | 30min | 60min | 120min |
| Human | Propantheline bromide | 2 | 100.0 | 84.0 | 66.8 | 53.4 | 26.4 | 7.2 |
| | Polypeptide compound 22 | 25 | 100.0 | 99.5 | 97.0 | 103.6 | 98.0 | 90.1 |
| | Polypeptide compound 22 + rt-PA | 25 | 100.0 | 97.9 | 95.8 | 102.8 | 91.3 | 95.6 |
| Rat | Lovastatin | 2 | 100.0 | 9.6 | 2.1 | 0.9 | NA | NA |
| | NA-1 | 25 | 100.0 | 103.7 | 77.5 | 69.5 | 54.4 | 42.1 |
| | NA-1 +rt-PA | 25 | 100.0 | 14.0 | 2.2 | 0.8 | NA | NA |
| | Polypeptide compound 22 | 25 | 100.0 | 116.5 | 105.4 | 109.7 | 101.9 | 124.8 |
| | Polypeptide compound 22 + rt-PA | 25 | 100.0 | 83.4 | 72.2 | 48.2 | 13.7 | 8.1 |

### 3.4.4. Conclusion

The results show that in human and rat plasma, within 2 h, NA-1 underwent significant degradation, and neither NoNO42 nor polypeptide compound 14 was degraded; after co-incubation with alteplase, the degradation of NA-1 was accelerated, polypeptide compound 14 still could remain stable in the plasma samples of two species, while the stability of NoNO42 in rat plasma was reduced but it remained stable in human plasma. The above results are consistent with the phase III clinical results of NA-1. The addition of alteplase will rapidly destroy the molecular structure of NA-1, causing it to lose its biological activity. The resistance of polypeptide compound 14 to alteplase in plasma makes it promising to become a molecule that overcomes the clinical disadvantages of NA-1.

Polypeptide compound 22 was stable in human and rat plasma. After alteplase was added, polypeptide compound 22 remained stable in human plasma, and its degradation rate in rat plasma was significantly slower than that of NA-1.

The resistance of polypeptide compounds 14 and 22 to alteplase in plasma enables them to overcome the clinical disadvantages of NA-1.

### 3.5. Pharmacokinetic study of polypeptide compounds in rats

### 3.5.1. Objective

With male SD rats as test animals, the pharmacokinetic behavior of polypeptide compounds administered via single intravenous bolus injection in rats (plasma) was studied.

### 3.5.2. Method

Male SD rats weighing 170 to 200 grams and aged 4-6 weeks were purchased from Beijing Vital River Laboratory Animal Technology Co., Ltd. The polypeptide compounds were dissolved in 1× PBS, and the polypeptide compound solutions were then administered via intravenous bolus injection over 10 min at a dose of 3 nmol/g, with three animals per group. At each of the time points of 2 min, 8 min, 15 min, 30 min, 45 min, 60 min, and 120 min after the end of bolus injection, 0.2 mL of blood was collected and transferred to an EDTA-K2 anticoagulation centrifuge tube. The whole blood sample was centrifuged at 4 °C at 4000 g for 5 min, and a plasma sample was obtained by separation and cryopreserved at -80 °C.

The pharmacokinetic parameters of polypeptide compound 22, administered via intravenous bolus injection, in rats were determined using a similar method, with the difference being that an enzyme inhibitor was added immediately after blood collection.

### 3.5.3. Sample analysis and data processing

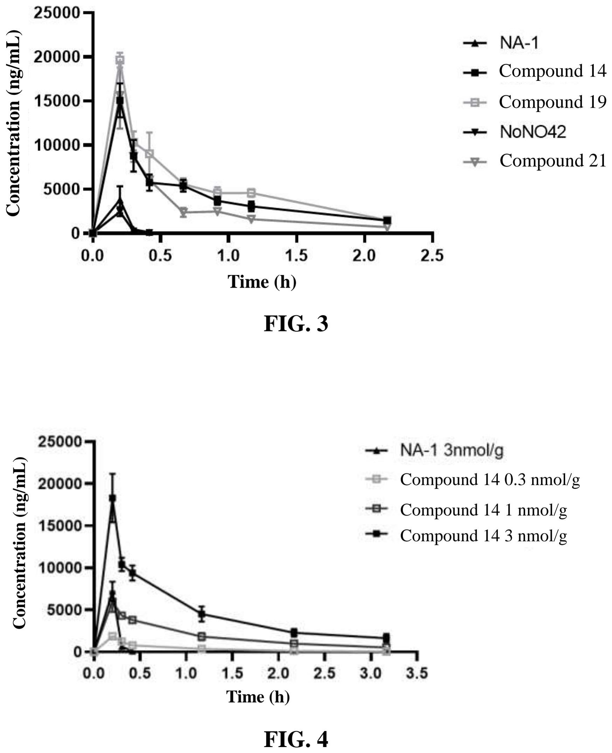

The plasma concentrations of the test compounds were determined using the method in Test Example 3.4.3. Plasma concentration-time curves were plotted, and the pharmacokinetic parameters were calculated using PKSolver software. The experimental results are shown in Table 5-1, Table 5-2, and FIG. 3.

### 3.5.4. Conclusion

The results in Table 5-1 and FIG. 3 show that the pharmacokinetic profiles of the positive drugs NA-1 and NoNO42 in rats were similar, with both exhibiting half-lives of less than 3 min. The pharmacokinetic profiles of the tested polypeptide compounds 14, 19, and 21 were significantly superior to those of the two positive drugs described above. Compared to NA-1, their half-lives significantly increased 18- to 23-fold, their areas under the curves (AUC) increased 18- to 33-fold, and their maximum plasma concentrations (Cmax) increased 4- to 5-fold. At the same concentration, polypeptide compounds 14, 19, and 21 exhibited longer duration of action and greater drug exposure.

The results in Table 5-2 show that the addition of the enzyme inhibitor caused a reduction in post-blood collection degradation of NA-1 and increases in both AUC and Cmax values. Compared to NA-1, the half-life of polypeptide compound 22 significantly increased 29-fold, its area under the curve (AUC) increased 19.4-fold, and its maximum plasma concentration (Cmax) increased 2.3-fold.

**Table 5-1. Intravenous bolus injection pharmacokinetic parameters in rats (n = 3)**

| Parameter | NA-1 | NoNO42 | Polypeptide compound 14 | Polypeptide compound 19 | Polypeptide compound 21 |
|---|---|---|---|---|---|
| AUC(0-t)_(h*ng/mL) | 309 | 191 | 7911 | 10221 | 5660 |
| AUC(inf)_(h*ng/mL) | 315 | 193 | 9902 | 11802 | 6396 |
| Cmax(ng/mL) | 3797 | 2450 | 15079 | 19631 | 15643 |
| T1/2(h) | 0.04 | 0.04 | 0.94 | 0.73 | 0.72 |
| AUClast/dose | 41 | 25 | 1041 | 1345 | 745 |
| MRTO-inf(h) | 0.06 | 0.05 | 1.19 | 0.96 | 0.80 |

**Table 5-2. Intravenous bolus injection pharmacokinetic parameters in rats (n = 3)**

| Parameter | NA-1 | Polypeptide compound 22 |
|---|---|---|
| AUC(inf)_(h*ng/mL) | 1248 | 24256 |
| Cmax(ng/mL) | 7250 | 16924 |
| T1/2(h) | 0.04 | 1.16 |
| Tmax(h) | 0.20 | 0.36 |
| AUClast/dose | 184.0 | 2239.6 |
| MRTO-inf(h) | 0.22 | 1.77 |

### 3.6. Pharmacokinetic study of different doses of polypeptide compound in rats

### 3.6.1. Objective

The neuroprotective agent NA-1 has demonstrated dose-related safety concerns in both preclinical and clinical studies. For example, at high doses, the agent causes symptoms such as tachycardia and blood pressure reduction due to excessive histamine release. Therefore, the pharmacokinetic profile of polypeptide compound 14 was assessed again using three doses: a high dose (3 nmol/g), a medium dose (1 nmol/g), and a low dose (0.3 nmol/g). In addition, a comparison was made with a high dose of NA-1 (3 nmol/g) to provide a dose reference for subsequent pharmacodynamic studies.

### 3.6.2. Method

The method of Test Examples 3.5.2 was used, with three animals per group and blood collection time points set as follows: 2 min, 8 min, 15 min, 60 min, 120 min, and 180 min after the end of intravenous bolus injection.

### 3.6.3. Sample analysis and data processing

The plasma concentrations of the test compounds were determined using the method in Test Example 3.4.3. Plasma concentration-time curves were plotted, and the pharmacokinetic parameters were calculated using WinNonlin software. The experimental results are shown in Table 6 and FIG. 4.

**Table 6. Intravenous bolus injection pharmacokinetic parameters in rats (different doses, n = 3)**

| Parameter | NA-1 (3 nmol/g) | Polypeptide compound 14 (0.3 nmol/g) | Polypeptide compound 14 (1 nmol/g) | Polypeptide compound 14 (3 nmol/g) |
|---|---|---|---|---|
| AUC(0-t)_(h*ng/mL) | 1149 | 1226 | 5857 | 14978 |
| AUC(inf)_(h*ng/mL) | 1154 | 1284 | 6603 | 17678 |
| Cmax(ng/mL) | 7188 | 1872 | 5973 | 18308 |
| T1/2(h) | 0.035 | 0.723 | 0.967 | 1.153 |
| MRTO-inf(h) | 0.125 | 0.856 | 1.307 | 1.474 |

### 3.6.4. Conclusion

As shown in Table 6, the plasma exposure of polypeptide compound 14 at the low dose was comparable to that of NA-1 at the high dose. Given that plasma exposure correlates with efficacy, it can be inferred that a 10-fold reduced dose of polypeptide compound 14 is likely to remain effective. The Cmax value is considered to correlate with histamine release. The Cmax values of polypeptide compound 14 at the medium and low doses were both lower than that of NA-1 at the high dose, suggesting a better safety profile. This was further verified in subsequent testing.

### 3.7. Testing of ability of polypeptide compound to induce histamine release

Histamine is an important endogenous component that mediates allergic and inflammatory responses. Drugs can induce mast cell degranulation, leading to massive histamine release and thus inflammatory symptoms such as redness, skin rash, blood pressure reduction, and heart rate reduction. The histamine-releasing effect of a drug is often evaluated using plasma histamine levels, which are typically determined by liquid chromatography-mass spectrometry.

### 3.7.1. Objective

With male SD rats as test animals, the ability of a polypeptide compound administered via single intravenous bolus injection to induce histamine release in rats (plasma) was studied.

### 3.7.2. Method

This test was performed synchronously with Test Examples 3.6, with three animals per group and blood collection time points set as follows: before administration, and 2 min, 8 min, and 15 min after the end of intravenous bolus injection.

### 3.7.3. Sample analysis and data processing

The histamine and 3-methylhistamine levels at each time point were determined by LC-MS/MS. LC-MS detection of compounds: (1) Chromatographic conditions: mobile phase A: acetonitrile/water/100 mM ammonium acetate, 50/45/5 (v/v/v), 2% FA; mobile phase B: acetonitrile/100 mM ammonium acetate, 95/5 (v/v), 2% FA; flow rate: 0.4 mL/min; chromatography column: Waters BEH HILIC 1.7 µm, 2.1 × 150 mm; column temperature: 50 °C; injection volume: 20 µL. (2) Mass-spectrometric conditions: The mass spectrometry analysis used an electrospray ionization (ESI) source, a positive ion analysis mode, and a multi-reaction monitoring (MRM) scan mode.

Sample pretreatment: A 30 µL plasma sample was taken and added to 200 µL of an acetonitrile solution containing tetradeuterated histamine (D4-histamine, 100 ng/mL). The mixture was vortexed for 1 min and then centrifuged at 5800 rpm for 10 min, and 100 µL of the supernatant was transferred to a sample tray and injected into an instrument for analysis. Table 7 shows sums of detected histamine and 3-methylhistamine.

**Table 7. Histamine levels at different time points**

| Time (min) | NA-1 (3 nmol/g) | Polypeptide compound 14 (0.3 nmol/g) | Polypeptide compound 14 (1 nmol/g) | Polypeptide compound 14 (3 nmol/g) |
|---|---|---|---|---|
| 0 | 21.0±5.9 | 34.1±21.7 | 45.5±14.4 | 50.2±28.7 |
| 2 | 390.7±164.2* | 18.2±5.2 | 41.9±23.3 | 754.7±448.6** |
| 8 | 86.6±36.6 | 38.7±16.8 | 59.4±53.5 | 558.0±405.6** |
| 15 | 48.3±19.2 | 45.3±3.3 | 71.4±38.7 | 265.3±145.7 |

| | | | | |
|---|---|---|---|---|
| Note: Compared to 0 min: *P < 0.05, and **P < 0.01. | | | | |

### 3.7.4. Conclusion

As can be seen from Table 7, histamine release after administration is a transient response of the organism to external stimulation. At the same high dose, both NA-1 and polypeptide compound 14 exhibited the strongest abilities to stimulate histamine release at 2 min: the histamine levels significantly differed from those before administration and then rapidly declined, and the peak release induced by polypeptide compound 14 was nearly twice that of NA-1. In contrast, no significant increase in histamine levels was detected within 15 min with either the medium or low dose of polypeptide compound 14. These results agree with the aforementioned pharmacokinetic results, indicating that the medium and low doses of polypeptide compound 14, due to their plasma concentrations being lower than that of the high dose of NA-1, have weaker abilities to induce histamine release than NA-1 and thus demonstrate higher safety.

### 3.8. Assessment of nephrotoxicity of polypeptide compound in rats

The kidney is an important target organ for drug toxicity, and nephrotoxicity is an important limiting factor in new drug development. Therefore, assessing potential nephrotoxicity is essential in the research and development pipeline of new drugs to reduce drug development risks. Blood urea nitrogen (BUN) and serum creatinine are important indicators for evaluating kidney function. Abnormal elevations in the two indicators in the blood indicate that there may be kidney function impairment or injury.

### 3.8.1. Objective

With male SD rats as test animals, the nephrotoxicity of a polypeptide compound administered via single intravenous bolus injection in rats (serum) was studied.

### 3.8.2. Method

This test was performed synchronously with Test Examples 3.6, with blood collection time points set as follows: before administration (day 0), and days 2, 4, and 7 post-administration. At each time point, 0.3 mL of blood was collected, transferred to a centrifuge tube, and left to stand at room temperature for 1 h. Then the whole blood sample was centrifuged at 4 °C at 4000 g for 5 min, and a serum sample was obtained by separation and cryopreserved at -80 °C.

### 3.8.3. Sample analysis and data processing

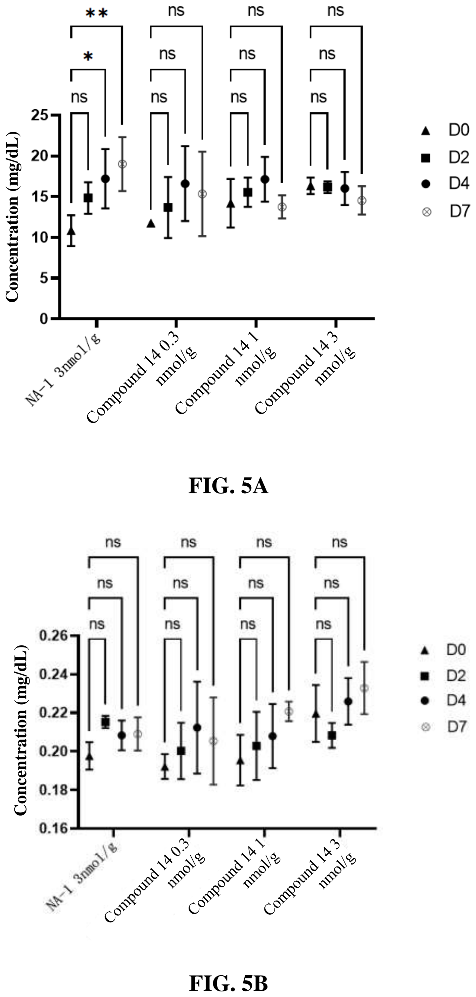

The blood urea nitrogen (BUN) and serum creatinine levels at each time point were determined through commercially available kits. For blood urea nitrogen, the urease continuous monitoring method was used. For serum creatinine, the sarcosine oxidase method was used. The sample was thawed in a refrigerator at 2 to 8 °C and vortexed for thorough mixing, and 100 µL of the sample was pipetted into a sample cup. The cup was placed in the sample position of a biochemistry analyzer, and assays were performed according to the instructions of the kits. The experimental results are shown in Tables 8-1 and 8-2, FIG. 5A, and FIG. 5B.

### 3.8.4. Conclusion

During the 7-day monitoring period, the blood urea nitrogen level of NA-1 significantly increased on day 4 and day 7 compared to before administration. However, the increases were within a 2-fold range, showing no clinical significance. Thus, these test results show that within the tested concentration range, neither NA-1 nor polypeptide compound 14 caused significant renal safety concerns.

**Table 8-1. Blood urea nitrogen (BUN) levels (in mg/dL) at different time points**

| Time (days) | NA-1 (3 nmol/g) | Polypeptide compound 14 (0.3 nmol/g) | Polypeptide compound 14 (1 nmol/g) | Polypeptide compound 14 (3 nmol/g) |
|---|---|---|---|---|
| 0 | 10.8±1.9 | 11.8±0.1 | 14.2±3.0 | 16.3±1.0 |
| 2 | 14.8±1.9 | 13.7±3.7 | 15.5±1.8 | 16.2±0.7 |
| 4 | 17.2±3.6* | 16.6±4.6 | 17.1±2.7 | 16.0±2.0 |
| 7 | 19±3.3** | 15.3±5.2 | 13.7±1.4 | 14.5±1.7 |

| | | | | |
|---|---|---|---|---|
| Note: Compared to before administration: *P < 0.05, and **P < 0.01. | | | | |

**Table 8-2. Serum creatinine (Crea) levels (in mg/dL) at different time points**

| Time (days) | NA-1 (3 nmol/g) | Polypeptide compound 14 (0.3 nmol/g) | Polypeptide compound 14 (1 nmol/g) | Polypeptide compound 14 (3 nmol/g) |
|---|---|---|---|---|
| 0 | 0.198±0.007 | 0.192±0.006 | 0.195±0.013 | 0.22±0.015 |
| 2 | 0.215±0.003 | 0.200±0.015 | 0.203±0.018 | 0.208±0.006 |
| 4 | 0.208±0.008 | 0.212±0.024 | 0.208±0.017 | 0.226±0.012 |
| 7 | 0.209±0.009 | 0.205±0.023 | 0.221±0.005 | 0.233±0.014 |

### 3.9. Pharmacokinetic study of polypeptide compound in beagle dogs

### 3.9.1. Objective

With standard beagle dogs as test animals, the pharmacokinetic behavior of a polypeptide compound administered via single intravenous infusion in beagle dogs (plasma) was studied.

### 3.9.2. Method

For polypeptide compound 22, two dose groups were set: 1.2 nmol/g and 0.4 nmol/g. For NA-1, one dose group was set: 1.2 nmol/g. Each group consisted of four animals, with an equal number of males and females. The polypeptide compound was dissolved in 1× PBS, and the polypeptide compound solution was then administered via intravenous infusion over 10 min. The blood collection time points were as follows: before infusion (0 h), 5 min after the start of bolus injection, and 0 min, 2 min, 5 min, 10 min, 15 min, 30 min, 1 h, 2 h, 4 h, 8 h, and 24 h after the end of bolus injection. At each time point, 0.5 mL of blood was collected and transferred to an EDTA-K2 anticoagulation centrifuge tube. Meanwhile, 50 µL of a protease inhibitor was added. After thorough mixing, the mixture was centrifuged at 2000 g for 10 min (4 °C), and the plasma was separated within 1 h, aliquoted into cryopreservation tubes, and stored at -60 to -90 °C. The blood collection-to-centrifugation process was performed under ice bath conditions.

### 3.9.3. Sample analysis and data processing

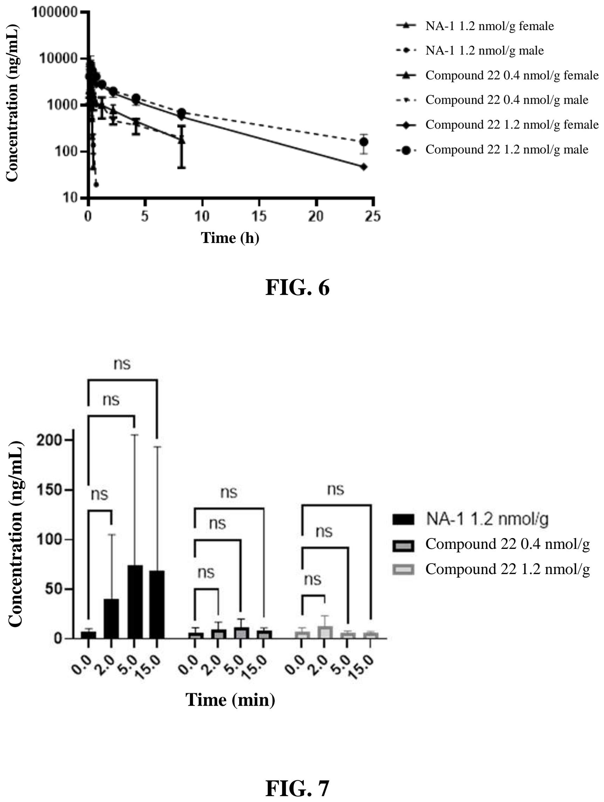

The plasma concentrations of the test compounds were determined using the method in Test Example 3.4.3. Plasma concentration-time curves were plotted, and the pharmacokinetic parameters were calculated using PKSolver software. The experimental results are shown in Table 9 and FIG. 6.

### 3.9.4. Conclusion

The results show that the pharmacokinetic profile of the tested polypeptide compound 22 was significantly superior to that of NA-1. At the same dose, the average T_{1/2} of NA-1 in dogs was 2.7 min, and the average T_{1/2} of polypeptide compound 22 in dogs was 5.1 h; the average AUC of polypeptide compound 22 was 21.3 times that of NA-1; and the average Cₘₐₓ of polypeptide compound 22 was 1.3 times that of NA-1. The drug exposure of polypeptide compound 22 was positively correlated with the dose administered.

**Table 9. Intravenous infusion pharmacokinetic parameters in beagle dogs**

| | NA-1 (1.2 nmol/g) | | Polypeptide compound 22 (0.4 nmol/g) | | Polypeptide compound 22 (1.2 nmol/g) | |
|---|---|---|---|---|---|---|
| Parameter | Female | Male | Female | Male | Female | Male |
| AUC(inf)_(h*ng/mL) | 855 | 1098 | 5886 | 5171 | 17995 | 23537 |
| Cmax(ng/mL) | 5028 | 6781 | 3930 | 2323 | 8131 | 7685 |
| T1/2(h) | 0.04 | 0.05 | 2.84 | 3.36 | 4.25 | 5.90 |
| Tmax(h) | 0.13 | 0.17 | 0.17 | 0.17 | 0.18 | 0.18 |
| AUClast/dose | 284 | 363 | 5730 | 4513 | 6558 | 8183 |
| MRTO-inf(h) | 0.15 | 0.16 | 3.66 | 4.61 | 4.91 | 7.03 |

### 3.10. Testing of ability of polypeptide compound to induce histamine release

### 3.10.1. Objective

With beagle dogs as test animals, the ability of a polypeptide compound administered via single intravenous infusion to induce histamine release in dogs (plasma) was studied.

### 3.10.2. Method

This test was performed synchronously with Test Examples 3.9, with four animals per group and blood collection time points set as follows: before administration (0 h), and 2 min, 5 min, and 15 min after the end of intravenous infusion.

### 3.10.3. Sample analysis and data processing

Sample analysis and data processing were performed with reference to the methods in 3.7.3. Table 10 and FIG. 7 show sums of detected histamine and 3-methylhistamine.

**Table 10. Histamine levels at different time points**

| Time (min) | NA-1 1.2 nmol/g | Polypeptide compound 22 0.4 nmol/g | Polypeptide compound 22 1.2 nmol/g |
|---|---|---|---|
| 0 | 7.08±3.15 | 6.28±5.01 | 7.06±4.14 |
| 2 | 40.47±64.51 | 9.45±7.38 | 12.71±10.47 |
| 5 | 73.95±131.85 | 11.70±8.39 | 6.24±1.78 |
| 15 | 68.80±124.89 | 7.97±2.75 | 5.81±1.44 |

### 3.10.4. Conclusion

Compared to before administration, polypeptide compound 22 exhibited no significant changes in histamine levels at both doses, indicating a good safety profile.

### 3.11. Study of pharmacodynamic effects of polypeptide compounds in rat tMCAO model

### 3.11.1. Objective

This study aims to study the pharmacodynamic effects of polypeptide compounds in a rat tMCAO (transient middle cerebral artery occlusion) model. A high dose of NA-1 (3 nmol/g) was used as a positive drug. A high (3 nmol/g) dose, a medium (1 nmol/g) dose, and a low (0.3 nmol/g) dose were set for polypeptide compounds 14 and 22. By comparing cerebral infarct sizes and neurological function scores in rats, along with the previous tests, this study provides safe and effective dose references for in-depth evaluation of polypeptide compounds 14 and 22.

### 3.11.2. Method

Modeling and administration: Male SD rats weighing 240 to 260 grams and aged 6-8 weeks were purchased from Beijing Vital River Laboratory Animal Technology Co., Ltd. The rat MCAO model was prepared using the intraluminal filament method. Administration was performed 60 min after a filament was inserted. After 90 min, the filament was removed, and reperfusion was performed. Before filament insertion, the baseline cerebral blood flow was monitored. The inclusion criteria were as follows: the cerebral blood flow is reduced by 50% or more compared to the baseline after filament insertion, and the cerebral blood flow is restored to 50% or more of the pre-reperfusion cerebral blood flow immediately after filament removal. Animals failing to meet the criteria were excluded. The polypeptide compounds were dissolved in normal saline to the required concentration. One hour after MCAO filament insertion, the polypeptide compound solutions were slowly administered via single tail vein bolus injection over 4 to 5 min. The model control group was given a 4- to 5-min intravenous injection of normal saline. The experimental endpoint was set to 24 h post-administration. During the administration period, the overall state of the test animals was observed: spontaneous locomotor activity, fasting and water intake, death, etc., and other abnormal behaviors.

Infarct size measurement: Twenty-four hours after MCAO, the surviving rats in each group were dissected. The hearts were perfused with pre-cooled PBS, decapitation was performed, and the whole brains were collected. Brain sections were stained in a 2% tetrazolium chloride (TTC) solution. The infarct size was calculated, and the percentage of infarct size was calculated. Percentage of cerebral infarct size = infarct size / total brain area × 100%.

Neurological function impairment scoring: Animals were subjected to blinded neurological function impairment scoring according to the scoring criteria in the table below before modeling (excluding animals with abnormal behavioral scores before modeling) and 24 h after administration. The total score was 16 points. Higher scores indicate more severe impairment. The scoring criteria are detailed in Table 11.

**Table 11. Scoring criteria for neurological function impairment in MCAO rats**

| Item | Performance | | Score |
|---|---|---|---|
| | 1) Lift the tail of the rat | | |
| | | Forelimb flexion | 1 |
| | | Hindlimb flexion | 1 |
| | | Head raised > 10. (with the vertical axis) within 30 s | 1 |
| 1. Motion tests | 2) Place the rat on the floor and let it walk (normal = 0; maximum = 3) | | |
| | | 0. Normally walks | 0 |
| | | 1. Fails to walk straight | 1 |
| | | 2. Circles toward the hemiplegic side | 2 |
| | | 3. Falls toward the hemiplegic side | 3 |
| 2. Beam balance test | | Can balance in a stable posture | 0 |
| | | Grasps the lateral sides of the beam | 1 |
| | | Can hold the beam, but one limb falls off from the beam | 2 |
| | | Can hold the beam, but two limbs fall off from the beam | 3 |
| | | Or spins on the beam for over 60 s | |
| | | Attempts to balance on the beam (>40 s), but falls off | 4 |
| | | Attempts to balance on the beam (>20 s), but falls off | 5 |
| | | Falls off, fails to balance, or does not want to struggle and hangs from the beam | 6 |
| 3. Reflex tests | | Auricular reflex (shakes its head when the entrance of the ear canal is touched) | 1 |
| | | Corneal reflex (blinks its eyes when the cornea is gently touched with cotton) | 1 |
| | | Startle reflex (produces a motor response to a brief paper-tearing sound) | 1 |
| | | Seizures, myoclonus, dystonia | 1 |
| Total | | | 16 |

### 3.11.3. Sample analysis and data processing

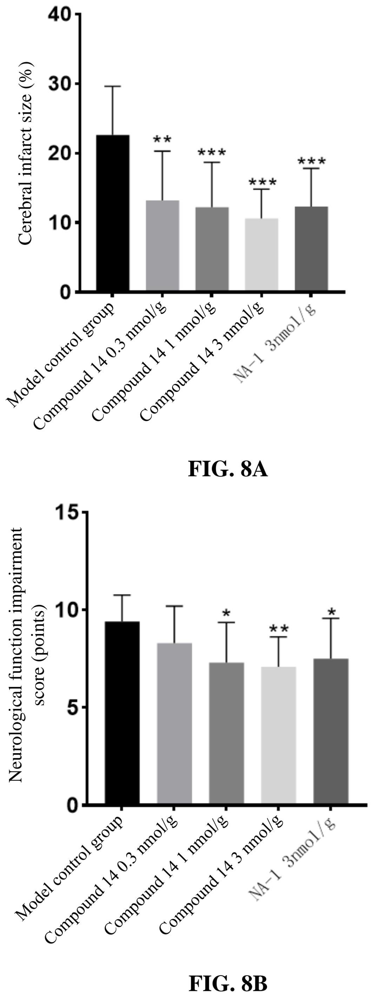

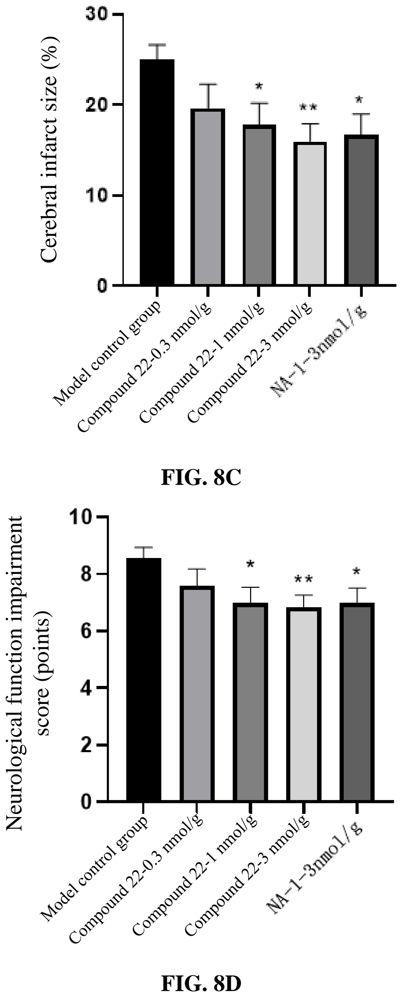

Data analysis was performed using IBM SPSS Statistics 25.0 statistical software. Test data are expressed as *x̅* ± s. LSD test was used for comparisons of data with homogeneous variance, and Dunnett's T3 test was used for data with heterogeneous variance. *P* < 0.05 indicates statistical significance. The specific experimental results are shown in Table 12-1, Table 12-2, FIG. 8A, FIG. 8B, FIG. 8C, and FIG. 8D.

**Table 12-1. A summary of pharmacodynamic results in the rat tMCAO model**

| Group | Polypeptide compound | Number of animals | Dose | Infarct size (%) | Infarct size improvement rate (%) | Neurological function score | Neurological function improvement rate (%) |
|---|---|---|---|---|---|---|---|
| 1 | Normal saline | 10 | / | 22.6±7.03 | - | 9.40±1.35 | - |
| 2 | NA-1 | 10 | 3 nmol/g | 12.3±5.53*** | 45.6 | 7.50±2.07* | 20.2 |
| 3 | 14 | 10 | 0.3 nmol/g | 13.2±7.08** | 41.6 | 8.30±1.89 | 11.7 |
| 4 | 14 | 10 | 1 nmol/g | 12.2±6.47*** | 46.0 | 7.30±2.06* | 22.3 |
| 5 | 14 | 10 | 3 nmol/g | 10.6±4.23*** | 53.1 | 7.10±1.52** | 24.5 |

| | | | | | | | |
|---|---|---|---|---|---|---|---|
| Note: Compared to the model group: *P < 0.05, **P < 0.01, and ***P < 0.001. | | | | | | | |

**Table 12-2. A summary of pharmacodynamic results in the rat tMCAO model**

| Group | Polypeptide compound | Number of animals | Dose | Infarct size (%) | Infarct size improvement rate (%) | Neurological function score | Neurological function improvement rate (%) |
|---|---|---|---|---|---|---|---|
| **1** | Normal saline | 7 | / | 24.98±4.31 | - | 8.57±0.98 | - |
| **2** | NA-1 | 10 | 3 nmol/g | 16.62±7.44* | 33.5 | 7±1.63* | 18.3 |
| **3** | 22 | 7 | 0.3 nmol/g | 19.64±6.93 | 21.4 | 7.57±1.62 | 11.7 |
| **4** | 22 | 7 | 1 nmol/g | 17.84±6.23* | 28.6 | 7±1.41* | 18.3 |
| **5** | 22 | 7 | 3 nmol/g | 15.97±5.16** | 36.1 | 6.86±1.07** | 20.0 |

### 3.11.4. Conclusion

The results show that polypeptide compounds 14 and 22 exhibited dose-dependent improving effects on both the neurological function and cerebral infarct size in tMCAO rats after single administration. The efficacy of polypeptide compounds 14 and 22 at the medium dose (1 nmol/g) was comparable to the efficacy of the positive drug NA-1 at the high dose (3 nmol/g).

## Claims

1. A compound represented by formula (I) or a pharmaceutically acceptable salt thereof:
CPP-L₁-X₁-X₂-X₃-X₄-X₅-X₆-X₇-X₈-X₉ (I)
wherein X₁ is selected from the group consisting of Gly and absent;
X₂ is selected from the group consisting of Aib, Ala, Ile, Cha, Phe, Trp, 1-Nal, Ser, Lys, Arg, Nle, Nva, Orn, cyclopropyl Ala, 4-thiazolyl Ala, homoLeu, and absent;
X₃ is selected from the group consisting of Dap, Dab, Thr, Aib, Ala, Cha, Phe, Trp, 1-Nal, Asn, Glu, Lys, Arg, 1Me-Trp, 2-Nal, Gln, Thr, 4-thiazolylalanine, and absent;
X₄ is selected from the group consisting of Thr, homoArg, Ser, and absent;
X₅ is selected from the group consisting of Tle, homoArg, and Chg;
X₆ is selected from the group consisting of Tle and Ile;
X₇ is selected from the group consisting of Thr and Ser;
X₈ is Asp;
X₉ is Val;
L₁ is a chemical bond or comprises polyethylene glycol in which one or two oxygen atoms are optionally replaced with a nitrogen atom;
CPP is an internalizing peptide.

2. The compound represented by formula (I) or the pharmaceutically acceptable salt thereof according to claim 1, wherein X₇ is Thr.

3. The compound represented by formula (I) or the pharmaceutically acceptable salt thereof according to any one of claims 1 to 2, wherein X₆ is Tle.

4. The compound represented by formula (I) or the pharmaceutically acceptable salt thereof according to any one of claims 1-3, wherein X₅ is Tle.

5. The compound represented by formula (I) or the pharmaceutically acceptable salt thereof according to claim 1, wherein X₆-X₇ is selected from the group consisting of Tle-Thr, Ile-Thr, and Tle-Ser, preferably from the group consisting of Tle-Thr and Ile-Thr, and most preferably from the group consisting of Tle-Thr.

6. The compound represented by formula (I) or the pharmaceutically acceptable salt thereof according to claim 1, wherein X₅-X₆ is selected from the group consisting of Tle-Tle, homoArg-Ile, and Chg-Tle, preferably from the group consisting of Tle-Tle and Chg-Tle, and most preferably from the group consisting of Tle-Tle.

7. The compound represented by formula (I) or the pharmaceutically acceptable salt thereof according to any one of claims 1 to 6, wherein X₅-X₆-X₇ is Tle-Tle-Thr.

8. The compound represented by formula (I) or the pharmaceutically acceptable salt thereof according to any one of claims 1 to 7, wherein one or more of X₁, X₂, X₃, and X₄ are optionally absent; preferably, X₂ and/or X₃ are/is absent, or X₁, X₂, X₃, and X₄ are all absent.

9. The compound represented by formula (I) or the pharmaceutically acceptable salt thereof according to any one of claims 1 to 8, wherein X₄ is selected from the group consisting of Thr, homoArg, and Ser, preferably from the group consisting of Thr and Ser, and most preferably from the group consisting of Thr.

10. The compound represented by formula (I) or the pharmaceutically acceptable salt thereof according to any one of claims 1 to 9, wherein X₃ is selected from the group consisting of Dap and Dab, preferably from the group consisting of Dab.

11. The compound represented by formula (I) or the pharmaceutically acceptable salt thereof according to any one of claims 1 to 10, wherein X₂ is selected from the group consisting of Aib, Ala, and Ile, preferably from the group consisting of Aib.

12. The compound represented by formula (I) or the pharmaceutically acceptable salt thereof according to any one of claims 1 to 11, wherein X₁ is selected from the group consisting of Nle, Gly, and absent, preferably from the group consisting of Gly.

13. The compound represented by formula (I) or the pharmaceutically acceptable salt thereof according to any one of claims 1 to 12, wherein L₁ is a chemical bond.

14. The compound represented by formula (I) or the pharmaceutically acceptable salt thereof according to any one of claims 1 to 12, wherein L₁ comprises polyethylene glycol in which one or two oxygen atoms are optionally replaced with a nitrogen atom, preferably AEEA.

15. The compound represented by formula (I) or the pharmaceutically acceptable salt thereof according to any one of claims 1 to 14, wherein X₁-X₂-X₃-X₄-X₅-X₆-X₇-X₈-X₉ is selected from the group consisting of any one of SEQ ID NO. 1 to SEQ ID NO. 8:
| | |
|---|---|
| SEQ ID NO. 1 | G-Aib-Dab-T-Tle-Tle-T-D-V |
| SEQ ID NO. 2 | Tle-Tle-T-D-V |
| SEQ ID NO. 3 | Nle-T-T-Tle-Tle-T-D-V |
| SEQ ID NO. 4 | T-homoArg-homoArg-I-T-D-V |
| SEQ ID NO. 5 | G-Aib-Dab-homoArg-Tle-Tle-T-D-V |
| SEQ ID NO. 6 | G-Aib-Dab-T-Chg-Tle-S-D-V |
| SEQ ID NO. 7 | G-T-Tle-Tle-T-D-V |
| SEQ ID NO. 8 | G-T-Chg-Tle-S-D-V. |

16. The compound represented by formula (I) or the pharmaceutically acceptable salt thereof according to any one of claims 1 to 15, wherein the internalizing peptide comprises the amino acid sequence set forth in any one of SEQ ID NO: 9 to SEQ ID NO. 11, preferably the amino acid sequence set forth in SEQ ID NO. 11; optionally, an amino acid residue in SEQ ID NO: 9 to SEQ ID NO. 11 is a D-amino acid:
| | |
|---|---|
| SEQ ID NO. 9 | RKKRRQRRR |
| SEQ ID NO. 10 | GRKKRRQRRR |
| SEQ ID NO. 11 | YGRKKRRQRRR. |

17. The compound represented by formula (I) or the pharmaceutically acceptable salt thereof according to claim 16, wherein:
1, 2, 3, 4, 5, 6, 7, 8, 9, 10, or 11 of the amino acid residues of the internalizing peptide are D-amino acids; preferably, 4, 5, 6, 7, 8, 9, 10, or 11 of the amino acid residues of the internalizing peptide are D-amino acids; most preferably, 9, 10, or 11 of the amino acid residues of the internalizing peptide are D-amino acids.

18. The compound represented by formula (I) or the pharmaceutically acceptable salt thereof according to claim 16, wherein:
an amino acid residue R of the internalizing peptide is a D-amino acid; preferably, 1, 2, 3, 4, 5, or 6 of the amino acid residues R are D-amino acids; preferably, 3, 4, 5, or 6 of the amino acid residues R are D-amino acids; most preferably, 5 or 6 of the amino acid residues R are D-amino acids.

19. The compound represented by formula (I) or the pharmaceutically acceptable salt thereof according to claim 16, wherein:
the first amino acid residue R from the C-terminus of the internalizing peptide is a D-amino acid, and the amino acid residue that is optionally 0, 1, 2, 3, 4, 5, 6, 7, 8, 9, or 10 amino acids apart from the first amino acid residue R is a D-amino acid;
preferably, the amino acid residue that is optionally 0, 1, 2, 3, 4, 5, 6, 7, or 8 amino acids apart from the first amino acid residue R is a D-amino acid; most preferably, the amino acid residue that is optionally 0, 1, 2, or 3 amino acids apart from the first amino acid residue R is a D-amino acid.

20. The compound represented by formula (I) or the pharmaceutically acceptable salt thereof according to any one of claims 16 to 19, wherein the internalizing peptide comprises the amino acid sequence set forth in SEQ ID NO: 12 or SEQ ID NO. 13:
| | |
|---|---|
| SEQ ID NO. 12 | y-G-r-k-k-r-r-q-r-r-r |
| SEQ ID NO. 13 | r-K-K-R-r-Q-R-R-r. |

21. The compound represented by formula (I) or the pharmaceutically acceptable salt thereof according to any one of claims 16 to 20, being selected from the group consisting of any one of SEQ ID NO. 14 to SEQ ID NO. 22:
| | |
|---|---|
| SEQ ID NO. 14 | y-G-r-k-k-r-r-q-r-r-r-G-Aib-Dab-T-Tle-Tle-T-D-V |
| SEQ ID NO. 15 | y-G-r-k-k-r-r-q-r-r-r-AEEA-Tle-Tle-T-D-V |
| SEQ ID NO. 16 | y-G-r-k-k-r-r-q-r-r-r-Nle-T-T-Tle-Tle-T-D-V |
| SEQ ID NO. 17 | y-G-r-k-k-r-r-q-r-r-r-T-homoArg-homoArg-I-T-D-V |
| SEQ ID NO. 18 | y-G-r-k-k-r-r-q-r-r-r-G-Aib-Dab-homoArg-Tle-Tle-T-D-V |
| SEQ ID NO. 19 | y-G-r-k-k-r-r-q-r-r-r-G-Aib-Dab-T-Chg-Tle-S-D-V |
| SEQ ID NO. 20 | y-G-r-k-k-r-r-q-r-r-r-G-T-Tle-Tle-T-D-V |
| SEQ ID NO. 21 | y-G-r-k-k-r-r-q-r-r-r-G-T-Chg-Tle-S-D-V |
| SEQ ID NO. 22 | r-K-K-R-r-Q-R-R-r-G-Aib-Dab-T-Tle-Tle-T-D-V. |

22. An active peptide or a pharmaceutically acceptable salt thereof, wherein the active peptide comprises formula (G):
X₁-X₂-X₃-X₄-X₅-X₆-X₇-X₈-X₉ (G),
wherein X₁, X₂, X₃, X₄, X₅, X₆, X₇, X₈, and X₉ are each as defined in any one of claims 1 to 12 or 15.

23. The active peptide or the pharmaceutically acceptable salt thereof according to claim 22, wherein the active peptide comprises the amino acid sequence set forth in any one of SEQ ID NO. 1 to SEQ ID NO. 8.

24. A peptide or a pharmaceutically acceptable salt thereof, comprising:
the active peptide defined in claim 22 or 23, and
the internalizing peptide defined in any one of claims 16 to 21.

25. The peptide or the pharmaceutically acceptable salt thereof according to claim 24, wherein the peptide comprises the amino acid sequence set forth in any one of SEQ ID NO. 14 to SEQ ID NO. 22.

26. A pharmaceutical composition, comprising:
the compound represented by formula (I) or the pharmaceutically acceptable salt thereof according to any one of claims 1 to 21, and/or the active peptide or the pharmaceutically acceptable salt thereof according to any one of claims 22 to 23, and/or the peptide or the pharmaceutically acceptable salt thereof according to any one of claims 24 to 25, and
a pharmaceutically acceptable carrier and/or excipient.

27. Use of the compound represented by formula (I) or the pharmaceutically acceptable salt thereof according to any one of claims 1 to 21, and/or the active peptide or the pharmaceutically acceptable salt thereof according to any one of claims 22 to 23, and/or the peptide or the pharmaceutically acceptable salt thereof according to any one of claims 24 to 25, and/or the pharmaceutical composition according to claim 26 in the manufacture of a medicament, wherein:
the medicament is used for treating and/or preventing strokes, cerebral ischemia, traumatic injury to the central nervous system, reperfusion injury, subarachnoid hemorrhage, concussion, pain, anxiety, epilepsy, neurodegenerative diseases, and/or diseases involving the risks described above.

28. Use of the compound represented by formula (I) or the pharmaceutically acceptable salt thereof according to any one of claims 1 to 21, and/or the active peptide or the pharmaceutically acceptable salt thereof according to any one of claims 22 to 23, and/or the peptide or the pharmaceutically acceptable salt thereof according to any one of claims 24 to 25, and/or the pharmaceutical composition according to claim 26 in combination with a thrombolytic drug in the manufacture of a medicament for treating and/or preventing acute ischemic strokes, wherein the thrombolytic drug is selected from the group consisting of urokinase, streptokinase, anistreplase, staphylokinase, recombinant staphylokinase, prourokinase, a *Desmodus rotundus* salivary plasminogen activator, lanoteplase, pamiteplase, monteplase, alteplase, reteplase, and tenecteplase; alteplase is preferred.
